(19) Europäisches Patentamt
European Patent Office
Office européen des brevets

(11) EP 0 971 959 B1

(12) EUROPEAN PATENT SPECIFICATION

(45) Date of publication and mention of the grant of the patent:
**28.12.2005 Bulletin 2005/52**

(21) Application number: **98918013.8**

(22) Date of filing: **03.04.1998**

(51) Int Cl.7: **C07K 16/00**

(86) International application number:
**PCT/US1998/006724**

(87) International publication number:
**WO 1998/045332 (15.10.1998 Gazette 1998/41)**

# (54) HUMANIZED ANTIBODIES AND METHODS FOR FORMING HUMANIZED ANTIBODIES

HUMANISIERTE ANTIKÖRPER UND VERFAHREN ZU IHRER HERSTELLUNG

ANTICORPS HUMANISES ET METHODE PERMETTANT DE LES PRODUIRE

(84) Designated Contracting States:
**AT BE CH CY DE DK ES FI FR GB GR IE IT LI LU MC NL PT SE**
Designated Extension States:
**AL LT LV MK RO SI**

(30) Priority: **07.04.1997 US 833504**

(43) Date of publication of application:
**19.01.2000 Bulletin 2000/03**

(73) Proprietor: **GENENTECH, INC.**
**South San Francisco, CA 94080-4990 (US)**

(72) Inventors:
- **WELLS, James, A.**
  **Burlingame, CA 94010 (US)**
- **BACA, Manuel**
  **Foster City, CA 94404 (US)**
- **PRESTA, Leonard, G.**
  **San Francisco, CA 94109 (US)**

(74) Representative: **Kiddle, Simon John et al**
**Mewburn Ellis LLP**
**York House,**
**23 Kingsway**
**London WC2B 6HP (GB)**

(56) References cited:
**WO-A-92/22653** **WO-A-94/04679**
**GB-A- 2 268 744** **US-A- 5 580 723**

- **KIM ET AL.,: "Inhibition of vascular endothelial growth factor-induced angiogenesis suppresses tumor growth in vivo" NATURE, vol. 362, 1993, page 841 XP002013864 London, GB cited in the application**
- **M.M. BENDIG: "Humanization of rodent monoclonal antibodies" METHODS: A COMPANION TO METHODS IN ENZYMOLOGY, vol. 8, 1995, pages 83-93, XP000647344 New York, NY, US**
- **M. BACA ET AL., : "Antibody humanization using monovalent phage display" JOURNAL OF BIOLOGICAL CHEMISTRY, vol. 272, no. 16, 18 April 1997, pages 10678-10684, XP002077471**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).



Printed by Jouve, 75001 PARIS (FR)

SEQUENCE LISTING

(1) GENERAL INFORMATION:

(i) APPLICANT: Genentech, Inc.

(ii) TITLE OF INVENTION: HUMANIZED ANTIBODIES AND METHODS FOR FORMING HUMANIZED ANTIBODIES

(iii) NUMBER OF SEQUENCES: 14

(iv) CORRESPONDENCE ADDRESS:

(A) ADDRESSEE: Flehr, Hohbach, Test, Albritton Herbert
(B) STREET: Four Embarcadero Center, Suite 3400
(C) CITY: San Francisco
(D) STATE: California
(E) COUNTRY: United States
(F) ZIP: 94111

(v) COMPUTER READABLE FORM:

(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30

(vi) CURRENT APPLICATION DATA:

(A) APPLICATION NUMBER: PCT HEREWITH
(B) FILING DATE: 02-APR-1998
(C) CLASSIFICATION:

(vii) PRIOR APPLICATION DATA:

(A) APPLICATION NUMBER:08/833,504
(B) FILING DATE: 07-APR-1997

(viii) ATTORNEY/AGENT INFORMATION:

(A) NAME: Dreger, Walter H.
(B) REGISTRATION NUMBER: 24,190
(C) REFERENCE/DOCKET NUMBER: A-64254

(ix) TELECOMMUNICATION INFORMATION:

(A) TELEPHONE: (415) 781-1989
(B) TELEFAX: (415) 398-3249

(2) INFORMATION FOR SEQ ID NO:1:

(i) SEQUENCE CHARACTERISTICS:

(A) LENGTH: 66 base pairs
(B) TYPE: nucleic acid
(C) STRANDEDNESS: unknown

(D) TOPOLOGY: unknown

(ii) MOLECULE TYPE: DNA (genomic)

(xi) SEQUENCE DESCRIPTION: SEQ ID NO:1:

GATTTCAAAC GTCGTNYTAC TWTTTCTTTA GACACCTCCG CAAGCACABY TTACCTGCAG 60

ATGAAC 66

(2) INFORMATION FOR SEQ ID NO:2:

(i) SEQUENCE CHARACTERISTICS:

(A) LENGTH: 60 base pairs
(B) TYPE: nucleic acid
(C) STRANDEDNESS: unknown
(D) TOPOLOGY: unknown

(ii) MOLECULE TYPE: DNA (genomic)

(xi) SEQUENCE DESCRIPTION: SEQ ID NO:2:

AGCCTGCGCG CTGAGGACAC TGCCGTCTAT TACTGTDYAA RGTACCCCCA CTATTATGGG 60

(2) INFORMATION FOR SEQ ID NO:3:

(i) SEQUENCE CHARACTERISTICS:

(A) LENGTH: 30 base pairs
(B) TYPE: nucleic acid
(C) STRANDEDNESS: unknown
(D) TOPOLOGY: unknown

(ii) MOLECULE TYPE: DNA (genomic)

(xi) SEQUENCE DESCRIPTION: SEQ ID NO:3:

CTCAGCGCGC AGGCTGTTCA TCTGCAGGTA 30

(2) INFORMATION FOR SEQ ID NO:4:

(i) SEQUENCE CHARACTERISTICS:

(A) LENGTH: 27 base pairs
(B) TYPE: nucleic acid
(C) STRANDEDNESS: unknown
(D) TOPOLOGY: unknown

(ii) MOLECULE TYPE: DNA (genomic)

(xi) SEQUENCE DESCRIPTION: SEQ ID NO:4:

TCTGGGACGG ATTACACTCT GACCATC 27

(2) INFORMATION FOR SEQ ID NO:5:

(i) SEQUENCE CHARACTERISTICS:

(A) LENGTH: 75 base pairs
(B) TYPE: nucleic acid
(C) STRANDEDNESS: unknown
(D) TOPOLOGY: unknown

(ii) MOLECULE TYPE: DNA (genomic)

(xi) SEQUENCE DESCRIPTION: SEQ ID NO:5:

CGTTTGTCCT GTGCARYTTC TGGCTATACC TTCACCAACT ATGGTATGAA CTGGRTCCGT 60

CAGGCCCCGG GTAAG 75

(2) INFORMATION FOR SEQ ID NO:6:

(i) SEQUENCE CHARACTERISTICS:

(A) LENGTH: 107 amino acids
(B) TYPE: amino acid
(C) STRANDEDNESS: unknown
(D) TOPOLOGY: unknown

(ii) MOLECULE TYPE: protein

(xi) SEQUENCE DESCRIPTION: SEQ ID NO:6:

```
Asp Ile Gln Met Thr Gln Thr Thr Ser Ser Leu Ser Ala Ser Leu Gly
1               5                   10                  15

Asp Arg Val Ile Ile Ser Cys Ser Ala Ser Gln Asp Ile Ser Asn Tyr
            20                  25                  30

Leu Asn Trp Tyr Gln Gln Lys Pro Asp Gly Thr Val Lys Val Leu Ile
        35                  40                  45

Tyr Phe Thr Ser Ser Leu His Ser Gly Val Pro Ser Arg Phe Ser Gly
    50                  55                  60

Ser Gly Ser Gly Thr Asp Tyr Ser Leu Thr Ile Ser Asn Leu Glu Pro
65                  70                  75                  80

Glu Asp Ile Ala Thr Tyr Tyr Cys Gln Gln Tyr Ser Thr Val Pro Trp
                85                  90                  95

Thr Phe Gly Gly Gly Thr Lys Leu Glu Ile Lys
            100                 105
```

(2) INFORMATION FOR SEQ ID NO:7:

(i) SEQUENCE CHARACTERISTICS:

(A) LENGTH: 107 amino acids
(B) TYPE: amino acid
(C) STRANDEDNESS: unknown
(D) TOPOLOGY: unknown

(ii) MOLECULE TYPE: protein

(xi) SEQUENCE DESCRIPTION: SEQ ID NO:7:

```
Asp Ile Gln Met Thr Gln Ser Pro Ser Ser Leu Ser Ala Ser Val Gly
1               5                   10                  15

Asp Arg Val Thr Ile Thr Cys Ser Ala Ser Gln Asp Ile Ser Asn Tyr
            20                  25                  30

Leu Asn Trp Tyr Gln Gln Lys Pro Gly Lys Ala Pro Lys Leu Leu Ile
        35                  40                  45

Tyr Phe Thr Ser Ser Leu His Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Ser Thr Val Pro Trp
                85                  90                  95

            Thr Phe Gly Gln Gly Thr Lys Val Glu Ile Lys
                        100                 105
```

(2) INFORMATION FOR SEQ ID NO:8:

(i) SEQUENCE CHARACTERISTICS:

(A) LENGTH: 107 amino acids
(B) TYPE: amino acid
(C) STRANDEDNESS: unknown
(D) TOPOLOGY: unknown

(ii) MOLECULE TYPE: protein

(xi) SEQUENCE DESCRIPTION: SEQ ID NO:8:

Asp Ile Gln Met Thr Gln Ser Pro Ser Ser Leu Ser Ala Ser Val Gly
1 5 10 15

Asp Arg Val Thr Ile Thr Cys Ser Ala Ser Gln Asp Ile Ser Asn Tyr
20 25 30

Leu Asn Trp Tyr Gln Gln Lys Pro Gly Lys Ala Pro Lys Leu Leu Ile
35 40 45

Tyr Phe Thr Ser Ser Leu His Ser Gly Val Pro Ser Arg Phe Ser Gly
50 55 60

Ser Gly Ser Gly Thr Asp Tyr Thr Leu Thr Ile Ser Ser Leu Gln Pro
65 70 75 80

Glu Asp Phe Ala Thr Tyr Tyr Cys Gln Gln Tyr Ser Thr Val Pro Trp
85 90 95

Thr Phe Gly Gln Gly Thr Lys Val Glu Ile Lys
100 105

(2) INFORMATION FOR SEQ ID NO:9:

(i) SEQUENCE CHARACTERISTICS:

(A) LENGTH: 123 amino acids
(B) TYPE: amino acid
(C) STRANDEDNESS: unknown
(D) TOPOLOGY: unknown

(ii) MOLECULE TYPE: protein

(xi) SEQUENCE DESCRIPTION: SEQ ID NO:9:

Glu Ile Gln Leu Val Gln Ser Gly Pro Glu Leu Lys Gln Pro Gly Glu
1 5 10 15

Thr Val Arg Ile Ser Cys Lys Ala Ser Gly Tyr Thr Phe Thr Asn Tyr
20 25 30

Gly Met Asn Trp Val Lys Gln Ala Pro Gly Lys Gly Leu Lys Trp Met
35 40 45

Gly Trp Ile Asn Thr Tyr Thr Gly Glu Pro Thr Tyr Ala Ala Asp Phe
50 55 60

Lys Arg Arg Phe Thr Phe Ser Leu Glu Thr Ser Ala Ser Thr Ala Tyr
65 70 75 80

```
Leu Gln Ile Ser Asn Leu Lys Asn Asp Asp Thr Ala Thr Tyr Phe Cys
                85                  90                  95

Ala Lys Tyr Pro His Tyr Tyr Gly Ser Ser His Trp Tyr Phe Asp Val
            100                 105                 110

Trp Gly Ala Gly Thr Thr Val Thr Val Ser Ser
        115                 120
```

(2) INFORMATION FOR SEQ ID NO:10:

(i) SEQUENCE CHARACTERISTICS:

(A) LENGTH: 123 amino acids
(B) TYPE: amino acid
(C) STRANDEDNESS: unknown
(D) TOPOLOGY: unknown

(ii) MOLECULE TYPE: protein

(xi) SEQUENCE DESCRIPTION: SEQ ID NO:10:

```
Glu Val Gln Leu Val Glu Ser Gly Gly Gly Leu Val Gln Pro Gly Gly
1               5                   10                  15

Ser Leu Arg Leu Ser Cys Ala Ala Ser Gly Tyr Thr Phe Thr Asn Tyr
            20                  25                  30

Gly Met Asn Trp Val Arg Gln Ala Pro Gly Lys Gly Leu Glu Trp Val
        35                  40                  45

Gly Trp Ile Asn Thr Tyr Thr Gly Glu Pro Thr Tyr Ala Ala Asp Phe
    50                  55                  60

Lys Arg Arg Phe Thr Ile Ser Arg Asp Asn Ser Lys Asn Thr Leu Tyr
65                  70                  75                      80

Leu Gln Met Asn Ser Leu Arg Ala Glu Asp Thr Ala Val Tyr Tyr Cys
                85                  90                  95

Ala Arg Tyr Pro His Tyr Tyr Gly Ser Ser His Trp Tyr Phe Asp Val
            100                 105                 110

Trp Gly Gln Gly Thr Leu Val Thr Val Ser Ser
        115                 120
```

(2) INFORMATION FOR SEQ ID NO:11:

(i) SEQUENCE CHARACTERISTICS:

(A) LENGTH: 123 amino acids
(B) TYPE: amino acid
(C) STRANDEDNESS: unknown
(D) TOPOLOGY: unknown

(ii) MOLECULE TYPE: protein

(xi) SEQUENCE DESCRIPTION: SEQ ID NO:11:

```
Glu Val Gln Leu Val Glu Ser Gly Gly Gly Leu Val Gln Pro Gly Gly
1               5                   10                  15

Ser Leu Arg Leu Ser Cys Ala Ala Ser Gly Tyr Thr Phe Thr Asn Tyr
            20                  25                  30

Gly Met Asn Trp Ile Arg Gln Ala Pro Gly Lys Gly Leu Glu Trp Val
        35                  40                  45

Gly Trp Ile Asn Thr Tyr Thr Gly Glu Pro Thr Tyr Ala Ala Asp Phe
    50                  55                  60

Lys Arg Arg Phe Thr Ile Ser Leu Asp Thr Ser Ala Ser Thr Val Tyr
65                  70                  75                  80

Leu Gln Met Asn Ser Leu Arg Ala Glu Asp Thr Ala Val Tyr Tyr Cys
                85                  90                  95

Ala Lys Tyr Pro His Tyr Tyr Gly Ser Ser His Trp Tyr Phe Asp Val
            100                 105                 110

Trp Gly Gln Gly Thr Ser Val Thr Val Ser Ser
        115                 120
```

(2) INFORMATION FOR SEQ ID NO:12:

(i) SEQUENCE CHARACTERISTICS:

(A) LENGTH: 66 base pairs
(B) TYPE: nucleic acid
(C) STRANDEDNESS: unknown
(D) TOPOLOGY: unknown

(ii) MOLECULE TYPE: DNA (genomic)

(xi) SEQUENCE DESCRIPTION: SEQ ID NO:12:

```
GATTTCAAAC GTCGTNYTAC TWTTTCTAGA GACAACTCCA AAAACACABY TTACCTGCAG   60

ATGAAC                                                              66
```

(2) INFORMATION FOR SEQ ID NO:13:

(i) SEQUENCE CHARACTERISTICS:

(A) LENGTH: 27 base pairs
(B) TYPE: nucleic acid
(C) STRANDEDNESS: unknown
(D) TOPOLOGY: unknown

(ii) MOLECULE TYPE: DNA (genomic)

(xi) SEQUENCE DESCRIPTION: SEQ ID NO:13:

GCTGATATCC AGTTGACCCA GTCCCCG 27

(2) INFORMATION FOR SEQ ID NO:14:

(i) SEQUENCE CHARACTERISTICS:

(A) LENGTH: 6072 base pairs
(B) TYPE: nucleic acid
(C) STRANDEDNESS: unknown
(D) TOPOLOGY: unknown

(ii) MOLECULE TYPE: DNA (genomic)

(ix) FEATURE:

(A) NAME/KEY: misc_feature
(B) LOCATION: 459..460
(D) OTHER INFORMATION: /note= "Light chain begins at base no. 459."

(ix) FEATURE:

(A) NAME/KEY: misc_feature
(B) LOCATION: 1101..1102
(D) OTHER INFORMATION: /note= "Light chain terminates at base no. 1101."

(ix) FEATURE:

(A) NAME/KEY: misc_feature
(B) LOCATION: 1254..1255
(D) OTHER INFORMATION: /note= "Heavy chain begins at base no. 1254."

(ix) FEATURE:

(A) NAME/KEY: misc_feature
(B) LOCATION: 2424..2425
(D) OTHER INFORMATION: /note= "Heavy chain terminates at base no. 2424."

(xi) SEQUENCE DESCRIPTION: SEQ ID NO:14:

```
GAATTCAACT TCTCCATACT TTGGATAAGG AAATACAGAC ATGAAAAATC TCATTGCTGA    60
GTTGTTATTT AAGCTTTGGA GATTATCGTC ACTGCAATGC TTCGCAATAT GGCGCAAAAT   120
GACCAACAGC GGTTGATTGA TCAGGTAGAG GGGGCGCTGT ACGAGGTAAA GCCCGATGCC   180
AGCATTCCTG ACGACGATAC GGAGCTGCTG CGCGATTACG TAAAGAAGTT ATTGAAGCAT   240
CCTCGTCAGT AAAAAGTTAA TCTTTTCAAC AGCTGTCATA AAGTTGTCAC GGCCGAGACT   300
TATAGTCGCT TTGTTTTTAT TTTTTAATGT ATTTGTAACT AGAATTCGAG CTCGGTACCC   360
GGGGATCCTC TAGAGGTTGA GGTGATTTTA TGAAAAAGAA TATCGCATTT CTTCTTGCAT   420
CTATGTTCGT TTTTTCTATT GCTACAAACG CGTACGCTGA TATCCAGATG ACCCAGTCCC   480
CGAGCTCCCT GTCCGCCTCT GTGGGCGATA GGGTCACCAT CACCTGCAGC GCAAGTCAGG   540
ATATTAGCAA CTATTTAAAC TGGTATCAAC AGAAACCAGG AAAAGCTCCG AAAGTACTGA   600
TTTACTTCAC CTCCTCTCTC CACTCTGGAG TCCCTTCTCG CTTCTCTGGA TCCGGTTCTG   660
GGACGGATTA CACTCTGACC ATCAGCAGTC TGCAGCCAGA AGACTTCGCA ACTTATTACT   720
GTCAACAGTA TAGCACCGTG CCGTGGACGT TTGGACAGGG TACCAAGGTG GAGATCAAAC   780
GAACTGTGGC TGCACCATCT GTCTTCATCT TCCCGCCATC TGATGAGCAG TTGAAATCTG   840
GAACTGCTTC TGTTGTGTGC CTGCTGAATA ACTTCTATCC CAGAGAGGCC AAAGTACAGT   900
GGAAGGTGGA TAACGCCCTC CAATCGGGTA ACTCCCAGGA GAGTGTCACA GAGCAGGACA   960
GCAAGGACAG CACCTACAGC CTCAGCAGCA CCCTGACGCT GAGCAAAGCA GACTACGAGA  1020
AACACAAAGT CTACGCCTGC GAAGTCACCC ATCAGGGCCT GAGCTCGCCC GTCACAAAGA  1080
GCTTCAACAG GGGAGAGTGT TAAGCTGATC CTCTACGCCG GACGCATCGT GGCCCTAGTA  1140
CGCAACTAGT CGTAAAAAGG GTATCTAGAG GTTGAGGTGA TTTTATGAAA AAGAATATCG  1200
CATTTCTTCT TGCATCTATG TTCGTTTTTT CTATTGCTAC AAACGCGTAC GCTGAGGTTC  1260
```

AGCTGGTGGA GTCTGGCGGT GGCCTGGTGC AGCCAGGGGG CTCACTCCGT TTGTCCTGTG 1320
CAGCTTCTGG CTATACCTTC ACCAACTATG GTATGAACTG GATCCGTCAG GCCCCGGGTA 1380
AGGGCCTGGA ATGGGTTGGA TGGATTAACA CCTATACCGG TGAACCGACC TATGCTGCGG 1440
ATTTCAAACG TCGTTTTACT ATTTCTTTAG ACACCTCCGC AAGCACAGTT TACCTGCAGA 1500
TGAACAGCCT GCGCGCTGAG GACACTGCCG TCTATTACTG TGCAAAGTAC CCCCACTATT 1560
ATGGGAGCAG CCACTGGTAT TTCGACGTCT GGGGTCAAGG AACCCTGGTC ACCGTCTCCT 1620
CGGCCTCCAC CAAGGGCCCA TCGGTCTTCC CCCTGGCACC CTCCTCCAAG AGCACCTCTG 1680
GGGGCACAGC GGCCCTGGGC TGCCTGGTCA AGGACTACTT CCCCGAACCG GTGACGGTGT 1740
CGTGGAACTC AGGCGCCCTG ACCAGCGGCG TGCACACCTT CCCGGCTGTC CTACAGTCCT 1800
CAGGACTCTA CTCCCTCAGC AGCGTGGTGA CCGTGCCCTC CAGCAGCTTG GGCACCCAGA 1860
CCTACATCTG CAACGTGAAT CACAAGCCCA GCAACACCAA GGTCGACAAG AAAGTTGAGC 1920
CCAAATCTTG TGACAAAACT CACCTCTAGA GTGGCGGTGG CTCTGGTTCC GGTGATTTTG 1980
ATTATGAAAA GATGGCAAAC GCTAATAAGG GGGCTATGAC CGAAAATGCC GATGAAAACG 2040
CGCTACAGTC TGACGCTAAA GGCAAACTTG ATTCTGTCGC TACTGATTAC GGTGCTGCTA 2100
TCGATGGTTT CATTGGTGAC GTTTCCGGCC TTGCTAATGG TAATGGTGCT ACTGGTGATT 2160
TTGCTGGCTC TAATTCCCAA ATGGCTCAAG TCGGTGACGG TGATAATTCA CCTTTAATGA 2220
ATAATTTCCG TCAATATTTA CCTTCCCTCC CTCAATCGGT TGAATGTCGC CCTTTTGTCT 2280
TTAGCGCTGG TAAACCATAT GAATTTTCTA TTGATTGTGA CAAAATAAAC TTATTCCGTG 2340
GTGTCTTTGC GTTTCTTTTA TATGTTGCCA CCTTTATGTA TGTATTTTCT ACGTTTGCTA 2400
ACATACTGCG TAATAAGGAG TCTTAATCAT GCCAGTTCTT TTGGCTAGCG CCGCCCTATA 2460
CCTTGTCTGC CTCCCCGCGT TGCGTCGCGG TGCATGGAGC CGGGCCACCT CGACCTGAAT 2520
GGAAGCCGGC GGCACCTCGC TAACGGATTC ACCACTCCAA GAATTGGAGC CAATCAATTC 2580
TTGCGGAGAA CTGTGAATGC GCAAACCAAC CCTTGGCAGA ACATATCCAT CGCGTCCGCC 2640
ATCTCCAGCA GCCGCACGCG GCGCATCTCG GGCAGCGTTG GGTCCTGGCC ACGGGTGCGC 2700
ATGATCGTGC TCCTGTCGTT GAGGACCCGG CTAGGCTGGC GGGGTTGCCT TACTGGTTAG 2760
CAGAATGAAT CACCGATACG CGAGCGAACG TGAAGCGACT GCTGCTGCAA AACGTCTGCG 2820
ACCTGAGCAA CAACATGAAT GGTCTTCGGT TTCCGTGTTT CGTAAAGTCT GGAAACGCGG 2880
AAGTCAGCGC CCTGCACCAT TATGTTCCGG ATCTGCATCG CAGGATGCTG CTGGCTACCC 2940
TGTGGAACAC CTACATCTGT ATTAACGAAG CGCTGGCATT GACCCTGAGT GATTTTTCTC 3000
TGGTCCCGCC GCATCCATAC CGCCAGTTGT TTACCCTCAC AACGTTCCAG TAACCGGGCA 3060
TGTTCATCAT CAGTAACCCG TATCGTGAGC ATCCTCTCTC GTTTCATCGG TATCATTACC 3120
CCCATGAACA GAAATTCCCC CTTACACGGA GGCATCAAGT GACCAAACAG GAAAAAACCG 3180

CCCTTAACAT GGCCCGCTTT ATCAGAAGCC AGACATTAAC GCTTCTGGAG AAACTCAACG 3240
AGCTGGACGC GGATGAACAG GCAGACATCT GTGAATCGCT TCACGACCAC GCTGATGAGC 3300
TTTACCGCAG GATCCGGAAA TTGTAAACGT TAATATTTTG TTAAAATTCG CGTTAAATTT 3360
TTGTTAAATC AGCTCATTTT TTAACCAATA GGCCGAAATC GGCAAAATCC CTTATAAATC 3420
AAAAGAATAG ACCGAGATAG GGTTGAGTGT TGTTCCAGTT TGGAACAAGA GTCCACTATT 3480
AAAGAACGTG GACTCCAACG TCAAAGGGCG AAAAACCGTC TATCAGGGCT ATGGCCCACT 3540
ACGTGAACCA TCACCCTAAT CAAGTTTTTT GGGGTCGAGG TGCCGTAAAG CACTAAATCG 3600
GAACCCTAAA GGGAGCCCCC GATTTAGAGC TTGACGGGGA AAGCCGGCGA ACGTGGCGAG 3660
AAAGGAAGGG AAGAAAGCGA AAGGAGCGGG CGCTAGGGCG CTGGCAAGTG TAGCGGTCAC 3720
GCTGCGCGTA ACCACCACAC CCGCCGCGCT TAATGCGCCG CTACAGGGCG CGTCCGGATC 3780
CTGCCTCGCG CGTTTCGGTG ATGACGGTGA AAACCTCTGA CACATGCAGC TCCCGGAGAC 3840
GGTCACAGCT TGTCTGTAAG CGGATGCCGG GAGCAGACAA GCCCGTCAGG GCGCGTCAGC 3900
GGGTGTTGGC GGGTGTCGGG GCGCAGCCAT GACCCAGTCA CGTAGCGATA GCGGAGTGTA 3960
TACTGGCTTA ACTATGCGGC ATCAGAGCAG ATTGTACTGA GAGTGCACCA TATGCGGTGT 4020
GAAATACCGC ACAGATGCGT AAGGAGAAAA TACCGCATCA GGCGCTCTTC CGCTTCCTCG 4080
CTCACTGACT CGCTGCGCTC GGTCGTTCGG CTGCGGCGAG CGGTATCAGC TCACTCAAAG 4140
GCGGTAATAC GGTTATCCAC AGAATCAGGG GATAACGCAG GAAAGAACAT GTGAGCAAAA 4200
GGCCAGCAAA AGGCCAGGAA CCGTAAAAAG GCCGCGTTGC TGGCGTTTTT CCATAGGCTC 4260
CGCCCCCCTG ACGAGCATCA CAAAAATCGA CGCTCAAGTC AGAGGTGGCG AAACCCGACA 4320
GGACTATAAA GATACCAGGC GTTTCCCCCT GGAAGCTCCC TCGTGCGCTC TCCTGTTCCG 4380
ACCCTGCCGC TTACCGGATA CCTGTCCGCC TTTCTCCCTT CGGGAAGCGT GGCGCTTTCT 4440
CATAGCTCAC GCTGTAGGTA TCTCAGTTCG GTGTAGGTCG TTCGCTCCAA GCTGGGCTGT 4500
GTGCACGAAC CCCCCGTTCA GCCCGACCGC TGCGCCTTAT CCGGTAACTA TCGTCTTGAG 4560
TCCAACCCGG TAAGACACGA CTTATCGCCA CTGGCAGCAG CCACTGGTAA CAGGATTAGC 4620
AGAGCGAGGT ATGTAGGCGG TGCTACAGAG TTCTTGAAGT GGTGGCCTAA CTACGGCTAC 4680
ACTAGAAGGA CAGTATTTGG TATCTGCGCT CTGCTGAAGC CAGTTACCTT CGGAAAAAGA 4740
GTTGGTAGCT CTTGATCCGG CAAACAAACC ACCGCTGGTA GCGGTGGTTT TTTTGTTTGC 4800
AAGCAGCAGA TTACGCGCAG AAAAAAAGGA TCTCAAGAAG ATCCTTTGAT CTTTTCTACG 4860
GGGTCTGACG CTCAGTGGAA CGAAAACTCA CGTTAAGGGA TTTTGGTCAT GAGATTATCA 4920
AAAAGGATCT TCACCTAGAT CCTTTTAAAT TAAAAATGAA GTTTTAAATC AATCTAAAGT 4980
ATATATGAGT AAACTTGGTC TGACAGTTAC CAATGCTTAA TCAGTGAGGC ACCTATCTCA 5040
GCGATCTGTC TATTTCGTTC ATCCATAGTT GCCTGACTCC CCGTCGTGTA GATAACTACG 5100

```
ATACGGGAGG GCTTACCATC TGGCCCCAGT GCTGCAATGA TACCGCGAGA CCCACGCTCA 5160
CCGGCTCCAG ATTTATCAGC AATAAACCAG CCAGCCGGAA GGGCCGAGCG CAGAAGTGGT 5220
CCTGCAACTT TATCCGCCTC CATCCAGTCT ATTAATTGTT GCCGGGAAGC TAGAGTAAGT 5280
AGTTCGCCAG TTAATAGTTT GCGCAACGTT GTTGCCATTG CTGCAGGCAT CGTGGTGTCA 5340
CGCTCGTCGT TTGGTATGGC TTCATTCAGC TCCGGTTCCC AACGATCAAG GCGAGTTACA 5400
TGATCCCCCA TGTTGTGCAA AAAAGCGGTT AGCTCCTTCG GTCCTCCGAT CGTTGTCAGA 5460
AGTAAGTTGG CCGCAGTGTT ATCACTCATG GTTATGGCAG CACTGCATAA TTCTCTTACT 5520
GTCATGCCAT CCGTAAGATG CTTTTCTGTG ACTGGTGAGT ACTCAACCAA GTCATTCTGA 5580
GAATAGTGTA TGCGGCGACC GAGTTGCTCT TGCCCGGCGT CAACACGGGA TAATACCGCG 5640
CCACATAGCA GAACTTTAAA AGTGCTCATC ATTGGAAAAC GTTCTTCGGG GCGAAAACTC 5700
TCAAGGATCT TACCGCTGTT GAGATCCAGT TCGATGTAAC CCACTCGTGC ACCCAACTGA 5760
TCTTCAGCAT CTTTTACTTT CACCAGCGTT TCTGGGTGAG CAAAAACAGG AAGGCAAAAT 5820
GCCGCAAAAA AGGGAATAAG GGCGACACGG AAATGTTGAA TACTCATACT CTTCCTTTTT 5880
CAATATTATT GAAGCATTTA TCAGGGTTAT TGTCTCATGA GCGGATACAT ATTTGAATGT 5940
ATTTAGAAAA ATAAACAAAT AGGGGTTCCG CGCACATTTC CCCGAAAAGT GCCACCTGAC 6000
GTCTAAGAAA CCATTATTAT CATGACATTA ACCTATAAAA ATAGGCGTAT CACGAGGCCC 6060
TTTCGTCTTC AA                                                     6072
```

## Description

### FIELD OF THE INVENTION

The present invention is directed at humanized antibodies and methods for preparing humanized antibodies. In particular, the present invention is directed at methods for preparing humanized antibodies using a monovalent phage display system and antibody mutants produced by random mutagenesis of a small set of critical framework residues made to a single human framework. More particularly, this invention is directed at the humanization of a murine antibody which binds to vascular endothelial growth factor (VEGF).

### BACKGROUND OF THE INVENTION

Monoclonal antibodies (mAbs) have enormous potential as therapeutic agents, particularly when they can be used to regulate defined systems. For example, in some circumstances it would be desirable to regulate a system such as angiogenesis, where new blood capillaries are formed from the walls of existing small vessels. Angiogenesis is generally important after infliction of a wound or infection so that a burst of capillary growth can be stimulated in the neighborhood of the damaged tissue. However, angiogenesis is also important in tumor growth since, for continued growth, a tumor must induce the formation of a capillary network that invades the tumor mass.

Certain growth factors have been identified which regulate angiogenesis. Of particular interest is the vascular endothelial growth factor (VEGF), which seems to be the agent by which some tumors acquire their rich blood supply. Molecular Biology of the Cell, 3rd Ed., Alberts et al., Garland Publishing, page 1154 (1994). Therefore, mAbs to VEGF, for example, can be useful for a variety of reasons, including for use in the regulation of angiogenesis and more particularly, as an anti-tumor agent. A murine anti-VEGF mAb A4.6.1 which blocks VEGF receptor binding has been previously described. This antibody has been shown to inhibit mitogenic signaling. Kim et al., *Growth Factors* 7, 53 (1992); Kim et al., *Nature* 362, 841 (1993).

Most mAbs including the anti-VEGF described above are derived from murine or other non-human sources which limits clinical efficacy. In particular, the body often reacts with an immunogenic response to non-human antibodies whereby the antibody is rapidly cleared from the system before any therapeutic effect can occur. In addition to the immunogenicity of non-human mAbs invoked when administered to humans, further limitations arise from weak recruitment of effector function.

As a means of circumventing these deficiencies, the antigen binding properties of non-human mAbs can be conferred to human antibodies through a process known as antibody "humanization". A humanized antibody contains the amino acid sequence from the six complementarity-determining regions (CDRs) (the antigen-binding site of the antibody molecule) of the parent or corresponding non-human mAb, grafted onto a human antibody framework. Therefore, humanization of non-human antibodies is commonly referred to as CDR grafting. The low content of non-human sequence in such humanized antibodies (~5%) has proven effective in reducing the immunogenicity and prolonging the serum half-life of the antibodies administered to humans. Inter alia, humanized monoclonal antibodies ("chimeric immunoglobulins") are disclosed in U.S. Patent No. 4,816,567.

Unfortunately, simple grafting of CDR sequences often yields humanized antibodies which bind antigen much more weakly than the parent non-human mAb. In order to restore high affinity, the antibody must be further engineered to fine-tune the structure of the antigen binding loops. This is achieved by replacing key residues in the framework regions of the antibody variable domains with the matching sequence from the parent murine antibody. These framework residues are usually involved in supporting the conformation of the CDR loops, although some framework residues may themselves directly contact the antigen. Studies have been conducted which note the importance of certain framework residues to CDR conformation and a comprehensive list of all the framework residues which can affect antigen binding has been compiled. Chothia et al., *J. Mol. Biol.* 224, 487 (1992); Foote et al., *J. Mol. Biol.* 224, 489 (1992). The comprehensive list includes some thirty "vernier" residues which can potentially contribute to CDR structure. Although higher antigen affinity would likely result from editing the entire set of vernier residues within a humanized antibody so as to match the corresponding parent non-human sequence, this is not generally desirable increased risk of immunogenicity imposed by adding further elements of non-human sequence. Thus, from a therapeutic standpoint, it is preferable to confine framework changes to the minimum set which affords a high affinity humanized antibody.

Therefore, it is desirable to identify a small set of changes which suffice to optimize binding, however, the required changes are expected to differ from one humanized antibody to the next. To achieve the desired result, one approach has been to identify the proper combination of mutations by constructing a panel of mutants having "suspect" framework residues replaced by their murine counterpart. These variants are each individually formed and tested for antigen and then combined with other variants found to have favorable binding affinities. However, this method involves cycles of individual site-directed mutagenesis, isolation and screening, and is therefore undesirable because it is time consuming and tedious.

As a means of simplifying antibody humanization, a number of different approaches have been developed. See, for example, Queen et al., *PNAS USA* 86, 10029 (1989); Kettleborough et al., *Protein Eng.* 4, 773 (1991); Tempest et al., *Biotechnology* 9, 266 (1991); Padlan, *Mol. Immunol.* 28, 489 (1991); Roguska et al., *PNAS USA* 91, 969 (1994); Studnicka et al., *Protein Eng.* 7, 805 (1994); Allen et al., *J. Immunol.* 135, 368 (1985); Carter et al., *PNAS USA* 89, 4285 (1992); Presta et al., *J. Immunol.* 151, 2623 (1993); Eigenbrot et al., *Proteins* 18, 49 (1994); Shalaby et al., *J. Exp. Med.* 175, 217 (1992); Kabat et al., Sequences of Proteins of Immunological Interest, (5th), Public Health Service, NIH, Bethesda, MD (1991); Rosok et al., *J. Biol. Chem.* 271, 22611 (1996); WO-A-92/22653, GB-A-2 268 744, and WO 94/04679. WO-A-92/22653 includes a list of preferred sites for mutation.

It is an object of the present invention to provide a general means of rapidly selecting framework mutations which improve the binding of humanized antibodies to their cognate antigens wherein the current methods of framework optimization based on cycles of individual site-directed mutagenesis and screening are eliminated.

It is also an object to provide rapid methods of humanizing antibodies which provide antibodies with low immunogenecity and which utilize a single human framework as a generic scaffold.

It is a further object of the present invention to provide humanized antibodies which are mutated to have enhanced affinity for antigen relative to the initial humanized antibody with no framework changes.

It is additionally a further object of the present invention to provide humanized antibodies that have a reduced clearance rate and hence longer retention within the body after systemic administration such that lower doses of the material are available for systemic administration for therapeutic effect.

It is also a further object of the present invention to provide humanized monoclonal antibodies to VEGF.

## SUMMARY OF THE INVENTION

The present invention provides a humanized antibody to vascular endothelial growth factor (VEGF), as defined in the claims. The initial humanized anti-VEGF has a framework derived from consensus sequences of the most abundant human subclasses, namely $V_L\kappa$ subgroup I ($V_I\kappa I$) and $V_H$ subgroup III ($V_HIII$) wherein the CDRs from non-human anti-VEGF are grafted thereon. Random mutagenesis of critical framework residues on the initial construct produced the humanized anti-VEGF described herein which has 125 fold enhanced affinity for antigen relative to the initial humanized antibody with no framework changes. A single additional mutation gave a further six fold improvement in binding. This humanized anti-VEGF can be reproduced by the method described herein or by traditional recombinant techniques given the sequence information provided herein.

Also disclosed herein is a method for rapidly producing and identifying framework mutations which improve the binding of humanized antibodies to their cognate antigens. Preferably, non-human CDRs are grafted onto a human $V_I\kappa I$- $V_HIII$ framework. Random mutagenesis of a small set of critical framework residues is also performed followed by monovalent display of the resultant library of antibody molecules on the surface of filamentous phage. The optimal framework sequences are then identified by affinity-based selection. Optionally, the selected antibodies can be further mutated so as to replace vernier residues which sit at the $V_L$-$V_H$ interface with residues which match the non-human parent antibody.

## BRIEF DESCRIPTION OF THE DRAWINGS

Figure 1 depicts the amino acid sequences of murine A4.6.1 (SEQ ID NO: 6 and 9 for the $V_L$ and $V_H$ domains, respectively), humanized A4.6.1 variant hu2.0, (SEQ ID NO: 7 and 10 for the $V_L$ and $V_H$ domains, respectively), and humanized A4.6.1 variant hu2.10 (SEQ ID NO: 8 and 11 for the $V_L$ and $V_H$ domains, respectively). Sequence numbering is according to Kabat et al., Sequences of Proteins of Immunological Interest, (5th), Public Health Service, NIH, Bethesda, MD (1991) and mismatches are indicated by asterisks (murine A4.6.1 vs hu2.0) or bullets (hu2.0 vs hu2.10). Variant hu2.0 contains only the CDR sequences (bold) from the murine antibody grafted onto a human light chain K subgroup I, heavy chain subgroup III framework. Variant hu2.10 is the consensus humanized clone obtained from phage sorting experiments described herein.

Figure 2 depicts the framework residues targeted for randomization.

Figure 3 depicts the phagemid construct for surface display of Fab-pIII fusions on phage. The phagemid construct encodes a humanized version of the Fab fragment for antibody A4.6.1 fused to a portion of the M13 gene III coat protein. The fusion protein consists of the Fab joined at the carboxyl terminus of the heavy chain to a single glutamine residue (from suppression of an amber codon in supE *E. coli),* then the C-terminal region of the gene III protein (residues 249-406). Transformation into $F^+$ *E. coli,* followed by superinfection with M13KO7 helper phage, produces phagemid particles in which a small proportion of these display a single copy of the fusion protein.

Detailed Description of the Invention:

A. Definitions

"Antibodies" (Abs) and "immunoglobulins" (Igs) are glycoproteins having the same structural characteristics. While antibodies exhibit binding specificity to a specific antigen, immunoglobulins include both antibodies and other antibody-like molecules which lack antigen specificity. Polypeptides of the latter kind are, for example, produced at low levels by the lymph system and at increased levels by myelomas.

"Native antibodies" and "native immunoglobulins" are usually heterotetrameric glycoproteins of about 150,000 daltons, composed of two identical light (L) chains and two identical heavy (H) chains. Each light chain is linked to a heavy chain by one covalent disulfide bond, while the number of disulfide linkages varies between the heavy chains of different immunoglobulin isotypes. Each heavy and light chain also has regularly spaced intrachain disulfide bridges. Each heavy chain has at one end a variable domain ($V_H$) followed by a number of constant domains. Each light chain has a variable domain at one and ($V_L$) and a constant domain at its other end; the constant domain of the light chain is aligned with the first constant domain of the heavy chain, and the light chain variable domain is aligned with the variable domain of the heavy chain. Particular amino acid residues are believed to form an interface between the light and heavy chain variable domains. Clothia et al., *J. Mol. Biol* 186, 651 (1985); Novotny et al., *PNAS USA* 82, 4592 (1985).

The term "variable" refers to the fact that certain portions of the variable domains differ extensively in sequence among antibodies and are used in the binding and specificity of each particular antibody for its particular antigen. However, the variability is not evenly distributed through the variable domains of antibodies. It is concentrated in three segments called "complementarity determining regions" (CDRs) or "hypervariable regions" both in the light chain and the heavy chain variable domains. The more highly conserved portions of variable domains are called the framework (FR). The variable domains of native heavy and light chains each comprise four FR regions, largely adopting a p-sheet configuration, connected by three CDRs, which form loops connecting, and in some cases forming part of, the β-sheet structure. The CDRs in each chain are held together in close proximity by the FR regions and, with the CDRs from the other chain, contribute to the formation of the antigen binding site of antibodies. Kabat et al., supra. The constant domains are not involved directly in binding an antibody to an antigen, but exhibit various effector functions, such as participation of the antibody in antibody-dependent cellular toxicity.

Papain digestion of antibodies produces two identical antigen binding fragments, called Fab fragments, each with a single antigen binding site, and a residual "Fc" fragment, whose name reflects its ability to crystallize readily. Pepsin treatment yields an $F(ab^1)_2$ fragment that has two antigen combining sites and is still capable of cross linking antigen.

"Fv" is the minimum antibody fragment which contains a complete antigen recognition and binding site. This region consists of a dimer of one heavy and one light chain variable domain in tight, non-covalent association. It is in this configuration that the three CDRs of each variable domain interact to define an antigen binding site on the surface of the $V_H$-$V_L$ dimer. Collectively, the six CDRs confer antigen binding specificity to the antibody. However, even a single variable domain (or half of an Fv comprising only three CDRs specific for an antigen) has the ability to recognize and bind antigen, although at a lower affinity than the entire binding site.

A "Fab" fragment contains the constant domain of the light chain and the first constant domain (CH1) of the heavy chain. Fab' fragments differ from Fab fragments by the addition of a few residues at the carboxy terminus of the heavy chain CH1 domain including one or more cysteines from the antibody hinge region. Fab'-SH is the designation herein for Fab' in which the cysteine residue(s) of the constant domains bear a free thiol group. $F(ab^1)_2$ antibody fragments originally were produced as pairs of $Fab^1$ fragments which have hinge cysteines between them. Other, chemical couplings of antibody fragments are also known.

The light chains of antibodies (immunoglobulins) from any vertebrate species can be assigned to one of two clearly distinct types, called kappa (κ) and lambda (λ), based on the amino acid sequences of their constant domains.

Depending on the amino acid sequence of the constant domain of their heavy chains, immunoglobulins can be assigned to different classes. There are five major classes of immunoglobulins: IgA, IgD, IgE, IgG and IgM, and several of these may be further divided into subclasses (isotypes), *e.g*. IgG-1, IgG-2, IgG-3, and IgG-4; IgA-1 and IgA-2. The heavy chain constant domains that correspond to the different classes of immunoglobulins are called α, delta, epsilon, γ, and, μ, respectively. The subunit structures and three-dimensional configurations of different classes of immunoglobulins are well known.

The term "antibody" is used in the broadest sense and specifically covers single monoclonal antibodies (including agonist and antagonist antibodies), antibody compositions with polyepitopic specificity, as well as antibody fragments (*e.g.,* Fab, $F(ab^1)_2$, and Fv), so long as they exhibit the desired biological activity.

The term "monoclonal antibody" as used herein refers to an antibody obtained from a population of substantially homogeneous antibodies, *i.e.*, the individual antibodies comprising the population are identical except for possible naturally occurring mutations that may be present in minor amounts. Monoclonal antibodies are highly specific, being directed against a single antigenic site. Furthermore, in contrast to conventional (polyclonal) antibody preparations which typically include different antibodies directed against different determinants (epitopes), each monoclonal antibody is directed against a single determinant on the antigen. In addition to their specificity, the monoclonal antibodies are advantageous in that they are synthesized by the hybridoma culture, uncontaminated by other immunoglobulins. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by the hybridoma method first described by Kohler et al., *Nature* 256, 495 (1975), or may be made by recombinant DNA methods, see, *e.g.* U.S. Patent No. 4,816,567.

"Chimeric" antibodies (immunoglobulins) are antibodies wherein a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity. U.S. Patent No. 4,816,567.

"Humanized" forms of non-human (*e.g.* murine) antibodies are chimeric immunoglobulins, immunoglobulin chains or fragments thereof (such as Fv, Fab, $Fab^1$, $F(ab^1)_2$ or other antigen-binding subsequences of antibodies) which contain minimal sequence derived from non-human immunoglobulin. For the most part, humanized antibodies are human immunoglobulins (recipient antibody) in which residues from a complementarity determining region (CDR) of the recipient are replaced by residues from a CDR of a non-human species (donor antibody) such as mouse, rat or rabbit having the desired specificity, affinity and capacity. In some instances, Fv framework residues of the human immunoglobulin are replaced by corresponding non-human residues. Furthermore, humanized antibody may comprise residues which are found neither in the recipient antibody nor in the imported CDR or framework sequences. These modifications are made to further refine and optimize antibody performance. In general, the humanized antibody will comprise substantially all of at least one, and typically two, variable domains, in which all or substantially all of the CDR regions correspond to those of a non-human immunoglobulin and all or substantially all of the FR regions are those of a human immunoglobulin consensus sequence. The humanized antibody optimally also will comprise at least a portion of an immunoglobulin constant region (Fc), typically that of a human immunoglobulin. For further details see: Jones et al., *Nature* 321, 522 (1986); Reichmann et al., *Nature* 332, 323 (1988); and Presta, *Curr. Op. Struct. Biol.* 2, 593 (1992).

"Non-immunogenic in a human" means that upon contacting the humanized antibody in a therapeutically effective amount with appropriate tissue of a human, a state of sensitivity or resistance to the humanized antibody is not substantially demonstratable upon administration.

As used herein, "vascular endothelial cell growth factor," or "VEGF," refers to a mammalian growth factor as defined in U.S. Patent 5,332,671, including the human amino acid sequence of Fig. 1. The biological activity of native VEGF is shared by any analogue or variant thereof that is capable of promoting selective growth of vascular endothelial cells but not of bovine corneal endothelial cells, lens epithelial cells, adrenal cortex cells, BHK-21 fibroblasts, or keratinocytes, or that possesses an immune epitope that is immunologically cross-reactive with an antibody raised against at least one epitope of the corresponding native VEGF.

"Site-directed mutagenesis" is a technique standard in the art, and is conducted using a synthetic oligonucleotide primer complementary to a single-stranded phage DNA to be mutagenized except for limited mismatching, representing the desired mutation. Briefly, the synthetic oligonucleotide is used as a primer to direct synthesis of a strand complementary to the phage, and the resulting double-stranded DNA is transformed into a phage-supporting host bacterium. Cultures of the transformed bacteria are plated in top agar, permitting plaque formation from single cells that harbor the phage. Theoretically, 50% of the new plaques will contain the phage having, as a single strand, the mutated form; 50% will have the original sequence. The plaques are hybridized with kinased synthetic primer at a temperature that permits hybridization of an exact match, but at which the mismatches with the original strand are sufficient to prevent hybridization. Plaques that hybridize with the probe are then selected and cultured, and the DNA is recovered.

"Expression system" refers to DNA sequences containing a desired coding sequence and control sequences in operable linkage, so that hosts transformed with these sequences are capable of producing the encoded proteins. To effect transformation, the expression system may be included on a vector; however, the relevant DNA may then also be integrated into the host chromosome.

As used herein, "cell," "cell line," and "cell culture" are used interchangeably and all such designations include progeny. Thus, "transformants" or "transformed cells" includes the primary subject cell and cultures derived therefrom without regard for the number of transfers. It is also understood that all progeny may not be precisely identical in DNA content, due to deliberate or inadvertent mutations. Mutant progeny that have the same functionality as screened for in the originally transformed cell are included. Where distinct designations are intended, it will be clear from the context.

"Plasmids" are designated by a lower case p preceded and/or followed by capital letters and/or numbers. The starting plasmids herein are commercially available, are publicly available on an unrestricted basis, or can be constructed from such available plasmids in accord with published procedures. In addition, other equivalent plasmids are known in the art and will be apparent to the ordinary artisan.

"Affinity binding" refers to the strength of the sum total of noncovalent interactions between a single antigen-binding site on an antibody and a single epitope. Low-affinity antibodies bind antigen weakly and tend to dissociate readily, whereas high-affinity antibodies bind antigen more tightly and remain bound longer.

"Transformation" means introducing DNA into an organism so that the DNA is replicable, either as an extrachromosomal element or by chromosomal integration. Depending on the host cell used, transformation is done using standard techniques appropriate to such cells. The calcium treatment employing calcium chloride, as described by Cohen, *Proc. Natl. Acad Sci. USA* 69, 2110 (1972) and Mandel et al., *J. Mol. Biol.* 53, 154 (1970), is generally used for prokaryotes or other cells that contain substantial cell-wall barriers. For mammalian cells without such cell walls, the calcium phosphate precipitation method of Graham and van der Eb, *Virology* 52, 456 (1978) is preferred. General aspects of mammalian cell host system transformations have been described by Axel in U.S. Pat. No. 4,399,216 issued August 16, 1983. Transformations into yeast are typically carried out according to the method of Van Solingen et al., *J. Bact.* 130, 946 (1977) and Hsiao et al., *Proc. Natl. Acad Sci. USA* 76, 3829 (1979). However, other methods for introducing DNA into cells such as by nuclear injection, electroporation or by protoplast fusion may also be used.

"Recovery" or "isolation" of a given fragment of DNA from a restriction digest means separation of the digest on polyacrylamide or agarose gel by electrophoresis, identification of the fragment of interest by comparison of its mobility versus that of marker DNA fragments of known molecular weight, removal of the gel section containing the desired fragment, and separation of the gel from DNA. This procedure is known generally. For example, see Lawn et al., *Nucleic Acids Res.* 9, 6103 (1981) and Goeddel et al., *Nucleic Acids Res.* 8, 4057 (1980).

"Ligation" refers to the process of forming phosphodiester bonds between two double stranded nucleic acid fragments. Unless otherwise provided, ligation may be accomplished using known buffers and conditions with 10 units of T4 DNA ligase ("ligase") per 0.5 mg of approximately equimolar amounts of the DNA fragments to be ligated.

The term "control sequences" refers to DNA sequences necessary for the expression of an operably linked coding sequence in a particular host organism. The control sequences that are suitable for prokaryotes, for example, include a promoter, optionally an operator sequence, a ribosome binding site, and possibly, other as yet poorly understood sequences. Eukaryotic cells are known to utilize promoters, polyadenylation signals, and enhancers.

Nucleic acid is "operably linked" or "operatively linked" when it is placed into a functional relationship with another nucleic acid sequence. For example, DNA for a presequence or a secretory leader is operably linked to DNA for a polypeptide if it is expressed as a preprotein that participates in the secretion of the polypeptide; a promoter or enhancer is operably linked to a coding sequence if it affects the transcription of the sequence; or a ribosome binding site is operably linked to a coding sequence if it is positioned so as to facilitate translation. Generally, "operably linked" or "operatively linked" means that the DNA sequences being linked are contiguous and, in the case of a secretory leader, contiguous and in reading phase. However, enhancers do not have to be contiguous. Linking is accomplished by ligation at convenient restriction sites. If such sites do not exist, then synthetic oligonucleotide adaptors or linkers are used in accord with conventional practice.

As used herein, "representatively numbered" refers to a position number of a residue in a particular sequence and corresponding position numbers in different sequences. Corresponding position numbers are those positions within sequences, generally human antibody framework sequences, which are functionally equivalent to the respresentatively numbered position when used in the construction of a humanized antibody.

Ordinarily, the terms "amino acid" and "amino acids" refer to all naturally occurring L-$\alpha$-amino acids. In some embodiments, however, D-amino acids may be present in the polypeptides or peptides of the present invention in order to facilitate conformational restriction. The amino acids are identified by either the single-letter or three-letter designations:

| | | | | | |
|---|---|---|---|---|---|
| Asp | D | aspartic acid | Ile | I | isoleucine |
| Thr | T | threonine | Leu | L | leucine |
| Ser | S | serine | Tyr | Y | tyrosine |
| Glu | E | glutamic acid | Phe | F | phenylalanine |
| Pro | P | proline | His | H | histidine |
| Gly | G | glycine | Lys | K | lysine |
| Ala | A | alanine | Arg | R | arginine |
| Cys | C | cysteine | Trp | W | tryptophan |

(continued)

| | | | | | |
|---|---|---|---|---|---|
| Val | V | valine | Gln | Q | glutamine |
| Met | M | methionine | Asn | N | asparagine |

The term "amino acid sequence variant" refers to molecules with some differences in their amino acid sequences as compared to a native amino acid sequence.

Substitutional variants are those that have at least one amino acid residue in a native sequence removed and a different amino acid inserted in its place at the same position. The substitutions may be single, where only one amino acid in the molecule has been substituted, or they may be multiple, where two or more amino acids have been substituted in the same molecule.

Hybridization is preferably performed under "stringent conditions" which means (1) employing low ionic strength and high temperature for washing, for example, 0.015 sodium chloride/0.0015 M sodium citrate/0.1% sodium dodecyl sulfate at 50°C, or (2) employing during hybridization a denaturing agent, such as formamide, for example, 50% (vol/vol) formamide with 0.1% bovine serum albumin/0.1% Ficoll/0.1% polyvinylpyrrolidone/50 nM sodium phosphate buffer at pH 6,5 with 750 mM sodium chloride, 75 mM sodium citrate at 42°C. Another example is use of 50% formamide, 5 x SSC (0.75 M NaCl, 0.075 M sodium citrate), 50 mM sodium phosphate (pH 6/8), 0.1% sodium pyrophosphate, 5 x Denhardt's solution, sonicated salmon sperm DNA (50 μg/ml), 0.1% SDS, and 10% dextran sulfate at 42°C, with washes at 42°C in 0.2 x SSC and 0.1% SDS. Yet another example is hybridization using a buffer of 10% dextran sulfate, 2 x SSC (sodium chloride/sodium citrate) and 50% formamide at 55°C, followed by a high-stringency wash consisting of 0.1 x SSC containing EDTA at 55°C. When a nucleic acid sequence of a nucleic acid molecule is provided, other nucleic acid molecules hybridizing thereto under the conditions described above are considered within the scope of the sequence.

Where amino acid sequences are described it is understood that these sequences can be reproduced by reconstructing the amino acid sequence synthetically or by mutation. Alternatively, it is understood that recombinant techniques can be used such that the DNA encoding the amino acid sequences is recovered. The DNA is recovered by forming a library from the DNA encoding the desired amino acid sequences. Probes are then generated based on the amino acid sequences. DNA hybridizing to the probes is then isolated and analyzed to determine whether the product encoded by the DNA is the desired product. Generally, cells are transformed with the DNA (or RNA) and expression studies are performed.

### B. <u>General Methodology for Humanizing Antibodies</u>

The methods described herein can be used to humanize any antibody. Similarly, it is understood that the humanized antibody specifically described herein, humanized anti-VEGF, can be reproduced by the methods described herein or by traditional DNA recombinant techniques. Specifically, since the critical framework residue mutations are described herein, the humanized antibody can be reproduced to have the same mutations without being reproduced using the monovalent phage display system. Rather, the DNA encoding the described amino acid sequences can be synthesized or reproduced by traditional DNA recombinant techniques. The DNA product can then be expressed, identified and recovered. Alternatively, site-directed mutagenesis can be performed on the antibody by methods known in the art, or the antibody can be synthesized so as to have the mutations described herein.

A particularly preferred method for producing the humanized antibodies described herein involves the following: preparing an antibody phagemid vector for monovalent display of Fab fragments having CDR sequences transplanted by site-directed mutagenesis onto a vector which codes for a human $V_L\kappa I\text{-}C\kappa_I$ light chain and human $V_HIII\text{-}C_HI\gamma$ heavy chain Fd; constructing the antibody Fab phagemid library by random mutagenesis of a small set of selected critical framework residues; expressing and purifying the humanized Fab fragments; selecting humanized Fab variants; and, determining binding affinities. These steps do not have to be performed in any particular order These steps are specifically described below in the "specific example" but are generally performed as follows:

*Preparation of antibody phagemid vector for monovalent display of Fab fragments*

First an antibody to be humanized is selected and the complementarity determining regions (CDRs) identified. The CDR sequences of the antibody can be identified according to the sequence definition of Kabat et al., supra. The CDR sequences are transplanted by site-directed mutagenesis onto a vector which codes for a human $V_L\kappa I\text{-}C\kappa_I$ light chain and human $V_HIII\text{-}C_HI\gamma_I$ heavy chain Fd. The Fab encoding sequence can then be subcloned into a phagemid vector. This construct encodes an initial humanized antibody wherein the C-terminus of the heavy chain is fused precisely to the carboxyl portion of a phage coat protein. Perferably, a phagemid vector is selected which provides expression of both secreted heavy chain or heavy chain-gene III fusions in *supE* suppressor strains of *E. coli.*

*Construction of the antibody Fab phagemid library*

Based on the cumulative results from humanizing a number of non-human antibodies onto a human $V_L\kappa I$-$V_H III$ framework, it was considered that framework changes required to optimize antigen binding are limited to some subset of the residues. See, Carter et al., *PNAS USA* 89, 4285 (1992); Presta et al., *J. Immunol.* 151, 2623 (1993); Eigenbrot et al., *Proteins* 18, 49-62 (1994); Shalaby et al., *J. Exp. Med.* 175, 217 (1992). Accordingly, a novel group of residues was selected for randomization. Randomizing these identified key framework residues provides the desired library of Fab variants to be displayed on the surface of filamentous phage. Specifically, $V_L$ residues 4 and 71 and $V_H$ residues 24, 37,67,69,71,73,75,76,78,93 and 94 have been selected as key framework residues important for antigen binding and targeted for randomization.

*Expression and purification of humanized Fab fragments*

Various methods are known in the art to express and purify fragments. As described herein, an *E. coli* strain 34B8, a nonsuppressor, was transformed with phagemid pMB419, or variants thereof. Single colonies were grown overnight at 37°C in 5 mL 2YT containing 50 μg/mL carbenicillin. These cultures were diluted into 200 mL AP5 medium, described in Chang et al., *Gene* 55, 189 (1987), containing 20 μg/mL carbenicillin and incubated for 26 hours at 30°C. The cells were pelleted at 4000 x g and frozen at -20°C for at least 2 hours. Cell pellets were then resuspended in 5 mL of 10 mM Tris-HCl (pH 7.6) containing 1 mM EDTA, shaken at 4°C for 90 minutes and centrifuged at 10,000 x g for 15 minutes. The supernatant was applied to a 1 mL streptococcal protein G-SEPHAROSE column (a column produced by Pharmacia) and washed with 10 mL of 10 mM MES (pH 5.5). The bound Fab fragment was eluted with 2.5 mL 100 mM acetic acid and immediately neutralized with 0.75 mL 1M TrisHCl, pH 8.0. Fab preparations were buffer-exchanged into PBS and concentrated using CENTRICON-30 concentrators (produced by Amicon). Typical yields ofFab were approximately 1 mg/L culture, post-protein G purification. Purified Fab samples were characterized by electrospray mass spectrometry, and concentrations were determined by amino acid analysis.

*Selection of humanized Fab variants*

Purified labeled antigen is coated onto a microtiter plate. The coating solution is discarded, the wells blocked, and phagemid stock is added. After a period, the wells are washed and the bound phage eluted and titered. The remaining phage eluted from the VEGF-coated well are propagated for use in the next selection cycle. This process can be repeated several times to obtain the desired number of clones. For example, a few dozen individual clones can be selected and sequenced.

*Determination of VEGF binding affinities*

Association and dissociation rate constants for binding of the humanized variants to VEGF are measured. Binding profiles are analyzed and those variants showing the highest affinities are selected.

*Administration of the humanized anti-VEGF*

Administration of the humanized anti-VEGF can be extrapolated from the data presented on the murine anti-VEGF described in Kim et al., *Growth Factors* 7, 53 (1992); Kim et al., *Nature* 362, 841 (1993). In particular, Kim et al. demonstrates that as little as 10 μg twice weekly of the VEGF antibody resulted in significant inhibition of tumor growth. Maximal effects were achieved with antibody doses of 50-100 μg.

The following example is intended merely to illustrate the best mode now known for practicing the invention but the invention is not to be considered as limited to the details of this example.

**Specific Example I**

<u>Construction of the phagemid vector and the initial humanized anti-VEGF</u>

The murine anti-VEGF mAb A4.6.1 has been previously described by Kim et al, *Growth Factors,* 7, 53 (1992); Kim et al., *Nature,* 362, 841 (1993). The first Fab variant of humanized A4.6.1, hu2.0, was constructed by site-directed mutagenesis using a deoxyuridine-containing template of plasmid pAK2 which codes for a human $V_L\kappa I$-$C\kappa_I$ light chain and human $V_H III$-$C_H I\gamma_I$ heavy chain Fd fragment. Carter et al., *PNAS USA* 89, 4285 (1992). The transplanted A4.6.1 CDR sequences were chosen according to the sequence definition of Kabat et al., Sequences of Proteins of Immunological Interest (5th), Public Health Service, National Institutes of Health, Bethesda, MD. (1991), except for CDR-H1 which we extended to encompass both sequence and structural definitions, viz $V_H$ residues 26-3 5, Chothia et al., *J. Mol. BioL* 196, 901 (1987). The Fab encoding sequence was subcloned into the phagemid vector phGHamg3. Bass and Wells, *Proteins,* 8, 309 (1990); Lowman et al., *Biochem.* 30, 10832 (1991). This construct, pMB4-19, encodes the initial humanized A4.6.1 Fab, hu2.0, with the C-terminus of the heavy chain fused precisely to the carboxyl portion of the M13 gene III coat protein. pMB4-19 is similar in construction to pDH188, a previously described plasmid for monovalent display of Fab fragments. Garrard et al., *Biotechn.* 9: 1373-1377 (1991). Notable differences between pMB4-19 and pDH188 include a shorter M13 gene III segment (codons 249-406) and use of an amber stop codon immediately following the antibody heavy chain Fd fragment. This permits expression of both secreted heavy chain or heavy chain-gene III fusions in *supE* suppressor strains of *E. coli.*

The initial humanized A4.6.1 Fab fragment (hu2.0) in which the CDRs from A4.6.1 were grafted onto a human $V_{L\kappa}$I-$V_H$III framework is shown in Figure 1. The $V_L$ domain of hu2.0 is set forth in SEQ ID NO: 7 and the $V_H$ domain of hu2.0 is set forth in SEQ ID NO: 10.

All residues other than the grafted CDRs were maintained as the human sequence. Binding of this initial humanized antibody to VEGF was so weak as to be undetectable. Based on the relative affinity of other weakly-binding humanized A4.6.1 variants (data not shown), the $K_D$ for binding of hu2.0 was estimated at >7 μM. This contrasts with an affinity of 1.6 nM for a chimeric Fab construct consisting of the intact $V_L$ and $V_H$ domains from murine A4.6.1 and human constant domains. Thus, binding of hu2.0 to VEGF was at least 4000-fold reduced relative to the chimera.

Design of the anti-VEGF Fab phagemid library

The group of framework changes required to optimize antigen binding when using human $V_L\kappa$I-$V_H$III framework were selected as shown in Table 1 and Figure 2. The humanized A4.6.1 phagemid library was constructed by site-directed mutagenesis according to the method of Kunkel et al., *Methods Enzymol.* 204, 125 (1991). A derivative of pMB4-19 containing TAA stop triplets at $V_H$ codons 24, 37, 67 and 93 was prepared for use as the mutagenesis template (all sequence numbering according to Kabat et al., supra. This modification was to prevent subsequent background contamination by wild type sequences. The codons targeted for randomization were 4 and 71 (light chain) and 24, 37, 67, 69, 71, 73, 75, 76, 78, 93 and 94 (heavy chain).

Table 1:

**Key framework residues important for antigen binding and targeted for randomization**

| Framework residue | | Human $V\kappa_L$I, $V_H$III consensus residue | Murine A4.6.1 residue | Randomization[a] |
|---|---|---|---|---|
| $V_L$ | 4 | Met | Met | Met,Leu |
| | 71 | Phe | Tyr | Phe, Tyr |
| $V_H$ | 24 | Ala | Ala | Ala, Val, Thr |
| | 37 | Val | Val | Val, Ile |
| | 67 | Phe | Phe | Phe, Val, Thr, Leu, Ile, Ala |
| | 69 | Ile | Phe | Ile, Phe |
| | 71 | Arg | Leu | Arg[b], Leu[b] |
| | 73 | Asp | Thr | Asp[b], Thr[b] |
| | 75 | Lys | Ala | Lys[b], Ala[b] |
| | 76 | Asn | Ser | Asn[b], Ser[b] |
| | 78 | Leu | Ala | Leu, Ala, Val, Phe |
| | 93 | Ala | Ala | Ala, Val, Leu, Ser, Thr |
| | 94 | Arg | Lys | Arg, Lys |

[a] Amino acid diversity in phagemid library

[b]$V_H$ 71, 73, 75, 76 randomized to yield the all-murine (L71/T73/A75/S76) or all-human (R71/D73/K75/N76) $V_H$III tetrad

A concern in designing the humanized A4.6.1 phagemid library was that residues targeted for randomization were widely distributed across the $V_L$ and $V_H$ sequences. Limitations in the length of synthetic oligonucleotides requires that simultaneous randomization of each of these framework positions can only be achieved through the use of multiple oligonucleotides. However, as the total number of oligonucleotides increases, the efficiency of mutagenesis decreases (*i.e.* the proportion of mutants obtained which incorporate sequence derived from all of the mutagenic oligonucleotides). To circumvent this problem, two features were incorporated into the library construction. The first was to prepare four different mutagenesis templates coding for each of the possible $V_L$ framework combinations. This was simple to do given the limited diversity of the light chain framework (only 4 different sequences), but was beneficial in that it eliminated the need for two oligonucleotides from the mutagenesis strategy. Secondly, two 126 base oligonucleotides were preassembled from smaller synthetic fragments. This made possible randomization of $V_H$ codons 67, 69, 71, 73, 75, 76, 93 and 94 with a single long oligonucleotide, rather than two smaller ones. The final randomization mutagenesis strategy therefore employed only two oligonucleotides simultaneously onto four different templates.

More specifically, in order to randomize heavy chain codons 67, 69, 71, 73, 75, 76, 78, 93 and 94 with a single mutagenic oligonucleotide, two 126-mer oligonucleotides were first preassembled from 60 and 66-mer fragments by template-assisted enzymatic ligation. Specifically, 1.5 nmol of 5' phosphorylated oligonucleotide GAT TTC AAA CGT CGT <u>NYT</u> ACT <u>WTT</u> TCT AGA GAC AAC TCC AAA AAC ACA <u>BYT</u> TAC CTG CAG ATG AAC (SEQ ID NO: 12) or GAT TTC AAA CGT CGT <u>NYT</u> ACT <u>WTT</u> TCT <u>TTA</u> GAC <u>ACC</u> TCC <u>GCA</u> <u>AGC</u> ACA <u>BYT</u> TAC CTG CAG ATG AAC (SEQ ID NO: 1) were combined with 1.5 nmol of AGC CTG CGC GCT GAG GAC ACT GCC GTC TAT TAC TGT <u>DYA</u> <u>ARG</u> TAC CCC CAC TAT TAT GGG (SEQ ID NO: 2). The randomized codons are underlined and N represents A/G/T/C; W represents A/T; B represents G/T/C; D represents G/A/T; R represents A/G; and Y represents C/T ("/" represents "or"). Then, 1.5 nmol of template oligonucleotide CTC AGC GCG CAG GCT GTT CAT CTG CAG GTA (SEQ ID NO: 3), with complementary sequence to the 5' ends of SEQ ID NOS: 12 and 1 and the 3' end of SEQ ID NO: 3 was added to hybridize to each end of the ligation junction. To this mixture, Taq ligase (thermostable ligase from New England Biolabs) and buffer were added, and the reaction mixture was subjected to 40 rounds of thermal cycling, (95 ° C for 1.25 minutes and 50°C for 5 minutes) so as to cycle the template oligonucleotide between ligated and unligated junctions. The product 126-mer oligonucleotides were purified on a 6% urea/TBE polyacrylamide gel and extracted from the polyacrylamide in buffer. The two 126-mer products were combined in equal ratio, ethanol precipitated and finally solubilized in 10 mM Tris-HCl, 1 mM EDTA. The mixed 126-mer oligonucleotide product was labeled 504-01.

Randomization of select framework codons ($V_L$ 4, 71; $V_H$ 24, 37, 67, 69, 71, 73, 75, 76, 93, 94) was thus effected in two steps. First, $V_L$ randomization was achieved by preparing three additional derivatives of the modified pMB4-19 template. Framework codons 4 and 71 in the light chain were replaced individually or pairwise using the two mutagenic oligonucleotides GCT GAT ATC CAG TTG ACC CAG TCC CCG (SEQ ID NO: 13) and TCT GGG ACG GAT <u>TAC</u> ACT CTG ACC ATC (SEQ ID NO: 4). Deoxyuridine containing template was prepared from each of these new derivatives. Together with the original template, these four constructs coded for each of the four possible light chain framework sequence combinations (see Table 1).

Oligonucleotides 504-01, the mixture of two 126-mer oligonucleotides, and CGT TTG TCC TGT GCA RYT TCT GGC TAT ACC TTC ACC AAC TAT GGT ATG AAC TGG <u>RTC</u> CGT CAG GCC CCG GGT AAG (SEQ ID NO: 5) were used to randomize heavy chain framework codons using each of the four templates just described. The four libraries were electroporated into *E. coli* XL-1 BLUE CELLS (marker cells produced by Stratagene) and combined. The total number of independent transformants was estimated at >1.2 x $10^8$, approximately 1,500-fold greater than the maximum number ofDNA sequences in the library.

From this strategy, each of residues 4 and 71 in the light chain and 24, 37, 67, 78 and 93 from the heavy chain were partially randomized to allow the selection of either the murine A4.6.1, human $V_L\kappa I$-$V_H III$ sequence, or sequences commonly found in other human and murine frameworks (Table I). Note that randomization of these residues was not confined to a choice between the human $V_L\kappa I$-$V_H III$ consensus or A4.6.1 framework sequences. Rather, inclusion of additional amino acids commonly found in other human and murine framework sequences allows for the possibility that additional diversity may lead to the selection of tighter binding variants.

Some of the heavy chain framework residues were randomized in a binary fashion according to the human $V_H III$ and murine A4.6.1 framework sequences. Residues $V_H$ 71, 73, 75 and 76 are positioned in a hairpin loop adjacent to the antigen binding site. The side chains of $V_H$ 71 and 73 are largely buried in canonical antibody structures and their potential role in shaping the conformation of CDR-H2 and CDR-H3 is well known. Kettleborough et al., *Protein Eng.* 4, 773 (1991); Carter et al., *PNAS USA* 89, 4285 (1992); Shalaby et al., *J. Exp. Med.* 175, 217 (1992). On the other hand, although the side chains of $V_H$ 75 and 76 are solvent exposed (Figure 2), it has nevertheless been observed that these two residues can also influence antigen binding (Eigenbrot, *Proteins* 18, 49 [1994]), presumably due to direct antigen contact in some antibody-antigen complexes. Because of their proximity in sequence and possible interdependence, $V_H$, 71, 73, 75 and 76 were randomized en bloc such that only two possible combinations of this tetrad could be selected; either all human $V_H III$ or all murine A4.6.1 sequence. Finally, $V_H$ residues 69 and 94 were randomized, but only to represent the $V_H III$ and A4.6.1 sequences. The $V_H$ 69 and 94 were not replaced in previous antibody humanizations, but because they differ between the $V_H III$ consensus and A4.6.1 sequences (Figure 1) and have been noted as potentially important for proper CDR conformation (Foote et al., *J. Mol. Biol.* 224, 487 [1992], they were included in this randomization strategy.

Humanized A4.6.1 Fab library displayed on the surface of phagemid

A variety of systems have been developed for the functional display of antibody fragments on the surface of filamentous phage. Winter et al., *Ann. Rev. Immunol.* 12, 433 (1994). These include the display of Fab or single chain Fv (scFv) fragments as fusions to either the gene III or gene VIII coat proteins of M13 bacteriophage. The system selected herein is similar to that described by Garrard et al., *Biotechn.* 9, 13 73 (1991) in which a Fab fragment is monovalently displayed as a gene III fusion (Figure 3). This system has two notable features. In particular, unlike scFvs, Fab fragments have no tendency to form dimeric species, the presence of which can prevent selection of the tightest binders due to avidity effects. Additionally, the monovalency of the displayed protein eliminates a second potential source of avidity effects that would otherwise result from the presence of multiple copies of a protein on each phagemid particle. Bass and Wells, *Proteins* 8, 309 (1990); Lowman et al., *Biochemistry* 30, 10832 (1991).

Phagemid particles displaying the humanized A4.6.1 Fab fragments were propagated in *E. coli* XL-1 Blue cells. Briefly, cells harboring the randomized pMB4-19 construct were grown overnight at 37°C in 25 mL 2YT medium containing 50 μg/mL carbenicillin and approximately $10^{10}$ M13KO7 helper phage (Viera and Messing, *Methods Enrymol.* 153, 3 [1987]). Phagemid stocks were purified from culture supernatants by precipitation with a saline polyethylene glycol solution, and resuspended in 100 μL PBS (approximately $10^{14}$ phagemid/mL).

Selection of humanized A4.6.1 Fab variants

Purified $VEGF_{121}$ (100 μL at 10 μg/mL in PBS) was coated onto a microtiter plate well overnight at 4°C. The coating solution was discarded and this well and an uncoated well were blocked with 6% skim milk for 1 hour and washed with PBS containing 0.05% TWEEN-20™(detergent). Then, 10 μL of phagemid stock, diluted to 100 μL with 20 mM Tris (pH 7.5) containing 0.1% BSA and 0.05% TWEEN-20™ was added to each well. After 2 hours, the wells were washed and the bound phage eluted with 100 μL of 0.1 M glycine (pH 2.0), and neutralized with 25 μL of 1M Tris pH 8.0. An aliquot of this was used to titer the number of phage eluted. The remaining phage eluted from the VEGF-coated well were propagated for use in the next selection cycle. A total of 8 rounds of selection was performed after which time 20 individual clones were selected and sequenced (Sanger et al., *PNAS USA* 74, 5463 [1977]).

Variants from the humanized A4.6.1 Fab phagemid library were thusly selected based on binding to VEGF. Enrichment of functional phagemid, as measured by comparing titers for phage eluted from a VEGF-coated versus uncoated microtiter plate well, increased up to the seventh round of affinity panning. After one additional round of sorting, 20 clones were sequenced to identify preferred framework residues selected at each position randomized. These results, summarized in Table 2, revealed strong consensus amongst the clones selected. Ten out of the twenty clones had the identical DNA sequence, designated hu2.10. Of the thirteen framework positions randomized, eight substitutions were selected in hu2.10 ($V_L$ 71; $V_H$ 37, 71, 73, 75, 76, 78 and 94). Interestingly, residues VH 37 (Ile) and 78 (Val) were selected neither as the human $V_H$III or murine A4.6.1 sequence. This result suggests that some framework positions may benefit from extending the diversity beyond the target human and parent murine framework sequences.

**Table 2: Sequences selected from the humanized A4.6.1 phagemid Fab library**

| Variant | Residue substitutions $V_L$ | | $V_H$ | | | | | | | | | | |
|---|---|---|---|---|---|---|---|---|---|---|---|---|---|
| | 4 | 71 | 24 | 37 | 67 | 69 | 71 | 73 | 75 | 76 | 78 | 93 | 94 |
| murine A4.6.1 | M | Y | A | V | F | F | L | T | A | S | A | A | K |
| hu2.0 (CDR-graft) | M | F | A | V | F | I | R | N | K | N | L | A | R |
| **Phage-selected clones:** | | | | | | | | | | | | | |
| hu2.1 (2) | - | Y | - | I | - | - | - | - | - | - | V | - | K |
| hu2.2 (2) | L | Y | - | I | - | - | - | - | - | - | V | - | K |
| hu2.6 (1) | L | - | - | I | T | - | L | T | A | S | V | - | K |
| hu2.7 (1) | L | - | - | I | T | - | - | - | - | - | V | - | K |
| hu2.10 (10) | - | Y | - | I | - | - | L | T | A | S | V | - | K |

Differences between hu2.0 and murine A4.6.1 antibodies are underlined. The number of identical clones identified for each phage-selected sequence is indicated in parentheses. Dashes in the sequences of phage-selected clones indicate selection of the human $V_L\kappa I$-$V_HIII$ framework sequence (i.e. as in hu2.0).

There were four other unique amino acid sequences among the remaining ten clones analyzed: hu2.1, hu2.2, hu2.6 and hu2.7. All of these clones, in addition to hu2.10, contained identical framework substitutions at positions $V_H$ 37 (Ile), 78 (Val) and 94 (Lys), but retained the human $V_H$III consensus sequence at positions 24 and 93. Four clones had lost the light chain coding sequence and did not bind VEGF when tested in a phage ELISA assay (Cunningham et al., *EMBO J.* 13, 2508 [1994]). We have occasionally noted the loss of heavy or light chain sequence with other Fab phagemid libraries (unpublished data), and these clones are presumably selected for on the basis of enhanced expression. Such artifacts can often be minimized by reducing the number of sorting cycles or by propagating libraries on solid media.

Determination of VEGF binding affinities

Association ($k_{on}$) and dissociation ($k_{off}$) rate constants for binding of humanized A4.6.1 Fab variants to $VEGF_{121}$ were measured by surface plasmon resonance (Karlsson et al, *J. Immun. Methods* 145, 229 [1991]) on a Pharmacia BIAcore instrument. $VEGF_{121}$ was covalently immobilized on the biosensor chip via primary amino groups. Binding of humanized A4.6.1 Fab variants was measured by flowing solutions of Fab in PBS/0.05% TWEEN-20™ (detergent) over the chip at a flow rate of 20 μL/min. Following each binding measurement, residual Fab was stripped from the immobilized ligand by washing with 5 μL of 50 mM aqueous HCl at 3 μL/min. Binding profiles were analyzed by nonlinear regression using a simple monovalent binding model (BIAevaluation software v2.0; Pharmacia).

Phage-selected variants hu2.1, hu2.2, hu2.6, hu2.7 and hu2.10 were expressed in *E. coli* using shake flasks and Fab fragments were purified from periplasmic extracts by protein G affinity chromatography. Recovered yields of Fab for these five clones ranged from 0.2 (hu2.6) to 1.7 mg/L (hu2.1). The affinity of each of these variants for antigen (VEGF) measured by surface plasmon resonance on a BIAcore instrument as shown in Table 3.

Table 3:

| VEGF binding affinity of humanized A4.6.1 Fab variants. | | | | |
|---|---|---|---|---|
| Variant | $k_{on}$ | $k_{off}$ | $K_D$ | $\frac{K_D(mut)}{K_D\ (A4.6.1)}$ |
| | $M^{-1}s^{-1}/10^4$ | $10^4 s^{-1}$ | nM | |
| A4.6.1 chimera | 5.4 | 0.85 | 1.6 | |
| hu2.0 | ND | ND | >7000** | >4000 |
| **Phage selected clones:** | | | | |
| hu2.1 | 0.70 | 18 | 260 | 170 |
| hu2.2 | 0.47 | 16 | 340 | 210 |
| hu2.6 | 0.67 | 4.5 | 67 | 40 |
| hu2.7 | 0.67 | 24 | 360 | 230 |
| hu2.10 | 0.63 | 3.5 | 55 | 35 |
| *hu2.10V | 2.0 | 1.8 | 9.3 | 5.8 |

*hu2.10V = hu2.10 with mutation $V_L$ Leu46 -> Val; Estimated errors in the Biacore binding measurements are +/- 25%;

**Too weak to measure, estimate of lower bound

Analysis of this binding data revealed that the consensus clone hu2.10 possessed the highest affinity for VEGF out of the five variants tested. Thus our Fab phagemid library was selectively enriched for the tightest binding clone. The calculated $K_D$ for hu2.10 was 55 nM, at least 125-fold tighter than for hu2.0- which contains no framework changes ($K_D$ >7 μM). The other four selected variants all exhibited weaker binding to VEGF, ranging down to a $K_D$ of 360 nM for the weakest (hu2.7). Interestingly, the $K_D$ for hu2.6, 67 nM, was only marginally weaker than that of hu2.10 and yet only one copy of this clone was found among 20 clones sequenced. This may have due to a lower level of expression and display, as was the case when expressing the soluble Fab of this variant. However, despite the lower expression rate, this variant is useful as a humanized antibody.

Additional improvement of humanized variant hu2.10

Despite the large improvement in antigen affinity over the initial humanized variant, binding of hu2.10 to VEGF was still 35-fold weaker than a chimeric Fab fragment containing the murine A4.6.1 $V_L$ and $V_H$ domains. This considerable difference suggested that further optimization of the humanized framework might be possible through additional mutations. Of the vernier residues identified by Foote et al., *J. Mol. Biol.* 196, 901 (1992), only residues $V_L$ 46, $V_H$ 2 and $V_H$ 48 differed in the A4.6.1 versus human $V_I\kappa$I-$V_H$III framework (Figure 1) but were not randomized in our phagemid library. A molecular model of the humanized A4.6.1 Fv fragment showed that $V_L$ 46 sits at the $V_L$-$V_H$ interface and could influence the conformation of CDRH3. Furthermore, this amino acid is almost always leucine in most $V_L\kappa$ frameworks (Kabat et al., supra.), but is valine in A4.6.1. Accordingly, a Leu -> Val substitution was made at this position in the background of hu2.10. Analysis of binding kinetics for this new variant, hu2. 10V, indicated a further 6-fold improvement in the $K_D$ for VEGF binding. The $K_D$ for hu2.10V (9.3 nM) was thus within 6-fold that of the chimera. In contrast to $V_L$ 46, no improvement in the binding affinity of hu2.10 was observed for replacement of either $V_H$ 2 or $V_H$ 48 with the corresponding residue from murine A4.6.1.

Interestingly, part of the improvement prior to the last change in affinity was due to an increase in the association rate constant ($k_{on}$), suggesting that $V_L$ 46 may play a role in preorganizing the antibody structure into a conformation more suitable for antigen binding. Other mutations which affected antigen affinity were primarily due to changes in the dissociation rate constant ($k_{off}$) for binding. Comparison of hu2.1 and hu2.10 reveals a 5-fold improvement in affinity for substitution of $V_H$ residues 71, 73, 75, 76 with the A4.6.1 sequence. Conversion of $V_L$- 71 to the A4.6.1 sequence (Phe -> Tyr) had negligible effect on binding (hu2.2 vs hu2.7), while variants with leucine at $V_L$ 4 bound marginally worse (<2-fold) than those with methionine, the naturally occurring residue in both the A4.6.1 and human $V_{KL}$I frameworks (hu2.2 vs hu2.1). Comparison of other humanized A4.6.1 variants not shown here revealed that the $V_H$ 94 Arg -> Lys change resulted in a 5-fold improvement in $K_D$, either due to direct antigen contact by this residue, or to a structural role in maintaining the proper conformation of CDR-H3. Variant hu2.6 has three sequence differences relative to the consensus clone hu2.10, but nevertheless has a similar $K_D$, thereby suggesting that these three substitutions have little effect on antigen binding. The negligible effect of conservative changes at $V_L$ 4 and 71 concurs with binding data for other variants, yet the change at $V_H$ 67 (Phe -> Thr) had little effect on binding.

## Claims

1. A humanized anti-vascular endothelial growth factor antibody, wherein the complementarity determining regions (CDRs) of a non-human antibody are grafted onto a human framework comprising the $V_L\kappa$ subgroup I ($V_L\kappa$I) framework of SEQ ID NO:7 and the $V_H$ subgroup III ($V_H$III) framework of SEQ ID NO:10, in which at least one of representatively numbered residues 4 and 71 of the $V_L$ domain is substituted with a different amino acid, and at least three of representatively numbered residues 37, 67, 69, 71, 73, 75, 76, 78 and 94 of the $V_H$ domain are substituted with a different amino acid, and wherein residue 46 of the $V_L$ domain is optionally substituted with the amino acid valine, and wherein the residue numbering is according to Kabat numbering as shown in Figure 1.

2. The humanized antibody of claim 1, having at least one of the following substitutions: at $V_L$4, leucine; and at $V_L$71, tyrosine; and at least three of the following substitutions: at $V_H$37, isoleucine; at $V_H$67, threonine; at $V_H$69, phenylalanine; at $V_H$71, leucine; at $V_H$73, threonine; at $V_H$75, alanine; at $V_H$76, serine; at $V_H$78, valine; and at $V_H$94, lysine.

3. The humanized antibody of claim 1 or claim 2, wherein residues 37, 78 and 94 of the $V_H$ domain are substituted with the amino acids isoleucine, valine and lysine, respectively.

4. The humanized antibody of any preceding claim, wherein residues 71, 73, 75 and 76 of the $V_H$ domain are substituted with the amino acids leucine, threonine, alanine and serine, respectively.

5. The humanized antibody of any preceding claim, wherein residue 46 of the $V_L$ domain is substituted with the amino acid valine.

6. The humanized antibody of any one of claims 1 to 4, wherein the $V_L$ domain has the sequence set forth in SEQ ID NO: 8 and the $V_H$ domain has the sequence set forth in SEQ ID NO: 11.

7. The humanized antibody of any one of claims 1 to 5, wherein:

the $V_L$ domain has the sequence set forth in SEQ ID NO: 8 in which residue 46, leucine, is substituted with valine and
the $V_H$ domain has the sequence set forth in SEQ ID NO: 11.

8. The humanized antibody of any one of claims 1 to 4, wherein:

the $V_L$ domain has the sequence set forth in SEQ ID NO: 8, in which residues 4, methionine, and 71, tyrosine, are substituted with leucine and phenylalanine, respectively; and
the $V_H$ domain has the sequence set forth in SEQ ID NO: 11, in which residue 67, phenylalanine, is substituted with threonine.

9. The humanized antibody of any one of claims 1 to 4, wherein:

the $V_L$ domain has the sequence set forth in SEQ ID NO: 7, in which residue 71, phenylalanine, is substituted with tyrosine; and
the $V_H$ domain has the sequence set forth in SEQ ID NO: 10, in which residues 37, valine; 78, leucine; and 94, arginine; are substituted with isoleucine, valine and lysine, respectively.

10. The humanized antibody of any one of claims 1 to 4, wherein:

the $V_L$ domain has the sequence set forth in SEQ ID NO: 7, in which residues 4, methionine and 71, phenylalanine, are substituted with leucine and tyrosine, respectively; and
the $V_H$ domain has the sequence set forth in SEQ ID NO: 10, in which residues 37, valine; 78, leucine; and 94, arginine; are substituted with isoleucine, valine and lysine, respectively.

11. The humanized antibody of any one of claims 1 to 4, wherein:

the $V_L$ domain has the sequence set forth in SEQ ID NO: 7, in which residue 4, methionine, is substituted with leucine; and
the $V_H$ domain has the sequence set forth in SEQ ID NO: 10, in which residues 37, valine; 67, phenylalanine; 78, leucine; and 94, arginine, a resubstituted with isoleucine, threonine, valine and lysine, respectively.

12. A method of humanizing a non-human anti-vascular endothelial growth factor antibody comprising the steps of:

grafting complementarity determining regions (CDRs) of a non-human antibody onto a human framework comprising the $V_L\kappa$ subgroup I ($V_L\kappa$I) and $V_H$ subgroup III ($V_H$III);
substituting at least one of residues 4 and 71 in the $V_L$ domain with a different amino acid; and
substituting at least three of residues 37, 67, 69, 71, 73, 75, 76, 78 and 94 in the $V_H$ domain with a different amino acid,

wherein the residue numbering is according to kabat numbering at shown in Figure 1.

13. The method of claim 12, wherein the human framework comprises, before substitution, the framework of the $V_L$K subgroup I ($V_L$KI) of SEQ ID NO:7 and the $V_H$ subgroup III ($V_H$III) of SEQ ID NO:10.

14. The method claim 12 or claim 13, comprising at least one of the following substitutions: at $V_L$4, leucine; and at $V_L$71, tyrosine; and at least three of the following substitutions: at $V_H$37, isoleucine; at $V_H$67, threonine; at $V_H$69, phenylalanine; at $V_H$71, leucine; at $V_H$73, threonine; at $V_H$75, alanine; at $V_H$76, serine; at $V_H$78, valine; and at $V_H$94, lysine.

15. The method of any one of claims 12 to 14, wherein residues 37, 78 and 94 of the $V_H$ domain are substituted with the amino acids isoleucine, valine and lysine, respectively.

**16.** The method of any one of claims 12 to 15, wherein residues 71, 73, 75 and 76 are substituted with the amino acids leucine, threonine, alanine and serine, respectively.

**17.** The method of any one of claims 12 to 16, wherein residue 46 of the $V_L$ domain is substituted with the amino acid valine.

**18.** The method of any one of claims 12 to 16, wherein the $V_L$ domain has the sequence set forth in SEQ ID NO: 8 and the $V_H$ domain has the sequence set forth in SEQ ID NO: 11.

**19.** The method of any one of claims 12 to 17, wherein:

the $V_L$ domain has the sequence set forth in SEQ ID NO: 8, in which residue 46, leucine, is substituted with valine; and
the $V_H$ domain has the sequence set forth in SEQ ID NO: 11.

**20.** The method of any one of claims 12 to 16, wherein:

the $V_L$ domain has the sequence set forth in SEQ ID NO: 8, in which residues 4, methionine, and 71, tyrosine, are substituted with leucine and phenylalanine, respectively; and
the $V_H$ domain has the sequence set forth in SEQ ID NO: 11, in which residue 67, phenylalanine, is substituted with threonine.

**21.** The method of any one of claims 12 to 15, wherein:

the $V_L$ domain has the sequence set forth in SEQ ID NO: 7, in which residue 71, phenylalanine, is substituted with tyrosine; and
the $V_H$ domain has the sequence set forth in SEQ ID NO: 10, in which residues 37, valine; 78, leucine; and 94, arginine; are substituted with isoleucine, valine and lysine, respectively.

**22.** The method of any one of claims 12 to 15, wherein wherein:

the $V_L$ domain has the sequence set forth in SEQ ID NO: 7, in which residues 4, methionine and 71, phenylalanine, are substituted with leucine and tyrosine, respectively; and
the $V_H$ domain has the sequence set forth in SEQ ID NO: 10, in which residues 37, valine; 78, leucine; and 94, arginine; are substituted with isoleucine, valine and lysine, respectively.

**23.** The method of any one of claims 12 to 15, wherein:

the $V_L$ domain has the sequence set forth in SEQ ID NO: 7, in which residue 4, methionine, is substituted with leucine; and
the $V_H$ domain has the sequence set forth in SEQ ID NO: 10, in which residues 37, valine; 67, phenylalanine; 78, leucine; and 94, arginine, are substituted with isoleucine, threonine, valine and lysine, respectively.

**24.** The method of any one of claims 12 to 17 further comprising the steps of:

displaying the $V_L$ and $V_H$ domains by substitutions on a phagemid;
determining whether VEGF will bind to the $V_L$ and $V_H$ domains by substitutions;
selecting humanized antibodies which will bind to VEGF.

**25.** The humanized antibody of any one of claims 1 to 11, which is an antibody fragment.

**26.** The antibody fragment of claim 25, which is a Fab, $Fab^1$, $F(ab^1)_2$ or Fv fragment.

**27.** The method of any one of claims 12 to 24, wherein the humanized antibody is an antibody fragment.

**28.** The method of claim 27, wherein the fragment is a Fab, $Fab^1$, $F(ab^1)_2$ or Fv fragment.

**29.** The humanized antibody of claim 1 encoded by a nucleic acid molecule having the sequence set forth in SEQ ID NO: 14.

**30.** Use of the humanized antibody of any one of claims 1 to 11, 25, 26 and 29 in the manufacture of a medicament for use in inhibiting tumour growth by inhibiting mitogenic signalling.

## Patentansprüche

**1.** Humanisierter Anti-Gefäßendothelwachstumsfaktor-Antikörper, worin die komplementaritätsbestimmenden Regionen (CDRs) eines nicht-menschlichen Antikörpers auf ein menschliches Gerüst aufgepfropft sind, das das $V_L$K-Untergruppen-I-($V_L$κI-) Gerüst aus Seq.-ID Nr. 7 und das $V_H$-Untergruppen-III- ($V_H$III-) Gerüst aus Seq.-ID Nr. 10 umfasst, worin zumindest einer der entsprechend nummerierten Reste 4 und 71 der $V_L$-Domäne durch eine unterschiedliche Aminosäure substituiert ist und zumindest drei der entsprechend nummerierten Reste 37, 67, 69, 71, 73, 75, 76, 78 und 94 der $V_H$-Domäne durch eine unterschiedliche Aminosäure substituiert sind und worin Rest 46 der $V_L$-Domäne gegebenenfalls durch die. Aminosäure Valin substituiert ist und worin die Nummerierung der Reste gemäß der Kabat-Nummerierung wie in Fig. 1 gezeigt gestaltet ist.

**2.** Humanisierter Antikörper nach Anspruch 1 mit zumindest einer der folgenden Substitutionen: an $V_L$4, Leucin; und an $V_L$71 , Tyrosin; und mit zumindest drei der folgenden Substitutionen: an $V_H$37, Isoleucin; an $V_H$67, Threonin; an $V_H$69, Phenylalanin; an $V_H$71, Leucin; an $V_H$73, Threonin; an $V_H$75, Alanin; an $V_H$76, Serin; an $V_H$78, Valin; und an $V_H$94, Lysin.

**3.** Humanisierter Antikörper nach Anspruch 1 oder Anspruch 2, worin die Reste 37, 78 und 94 der $V_H$-Domäne durch die Aminosäuren Isoleucin, Valin bzw. Lysin substituiert sind.

**4.** Humanisierter Antikörper nach einem der vorangehenden Ansprüche, worin die Reste 71, 73, 75 und 76 der $V_H$-Domäne durch die Aminosäuren Leucin, Threonin, Alanin bzw. Serin substituiert sind.

**5.** Humanisierter Antikörper nach einem der vorangehenden Ansprüche, worin Rest 46 der $V_L$-Domäne durch die Aminosäure Valin substituiert ist.

**6.** Humanisierter Antikörper nach einem der Ansprüche 1 bis 4, worin die $V_L$-Domäne die in Seq.-ID Nr. 8 gezeigte Sequenz aufweist und die $V_H$-Domäne die in Seq.-ID Nr. 11 gezeigte Sequenz aufweist.

**7.** Humanisierter Antikörper nach einem der Ansprüche 1 bis 5, worin:

die $V_L$-Domäne die in Seq.-ID Nr. 8 gezeigte Sequenz aufweist, in der Rest 46, Leucin, durch Valin substituiert ist, und
die $V_H$-Domäne die in Seq.-ID Nr. 11 gezeigte Sequenz aufweist.

**8.** Humanisierter Antikörper nach einem der Ansprüche 1 bis 4, worin:

die $V_L$-Domäne die in Seq.-ID-Nr. 8 gezeigte Sequenz aufweist, in der die Reste 4, Methionin, und 71, Tyrosin, durch Leucin bzw. Phenylalanin substituiert sind; und
die $V_H$-Domäne die in Seq.-ID Nr. 11 gezeigte Sequenz aufweist, in der Rest 67, Phenylalanin, durch Threonin substituiert ist.

**9.** Humanisierter Antikörper nach einem der Ansprüche 1 bis 4, worin:

die $V_L$-Domäne die in Seq.-ID Nr. 7 gezeigte Sequenz aufweist, in der Rest 71, Phenylalanin, durch Tyrosin substituiert ist; und
die $V_H$-Domäne die in Seq.-ID Nr. 10 gezeigte Sequenz aufweist, in der die Reste 37, Valin; 78, Leucin; und 94, Arginin; durch Isoleucin, Valin bzw. Lysin substituiert sind.

**10.** Humanisierter Antikörper nach einem der Ansprüche 1 bis 4, worin:

die $V_L$-Domäne die in Seq.-ID Nr. 7 gezeigte Sequenz aufweist, in der die Reste 4, Methionin, und 71, Phenylalanin, durch Leucin bzw. Tyrosin substituiert sind; und

die $V_H$-Domäne die in Seq.-ID Nr. 10 gezeigte Sequenz aufweist, in der die Reste 37, Valin; 78, Leucin; und 94, Arginin; durch Isoleucin, Valin bzw. Lysin substituiert sind.

**11.** Humanisierter Antikörper nach einem der Ansprüche 1 bis 4, worin:

die $V_L$-Domäne die in Seq.-ID Nr. 7 gezeigte Sequenz aufweist, in der Rest 4, Methionin, durch Leucin substituiert ist; und
die $V_H$-Domäne die in Seq.-ID Nr. 10 gezeigte Sequenz aufweist, in der die Reste 37, Valin; 67, Phenylalanin; 78, Leucin; und 94, Arginin; durch Isoleucin, Threonin, Valin bzw. Lysin substituiert sind.

**12.** Verfahren zur Humanisierung eines nicht-menschlichen Anti-Gefäßendothelwachstumsfaktor-Antikörpers, die folgenden Schritte umfassend:

das Aufpfropfen von komplementaritätsbestimmenden Regionen (CDRs) eines nicht-menschlichen Antikörpers auf ein menschliches Gerüst, das die $V_L$K-Untergruppe I ($V_L$KI) und die $V_H$-Untergruppe III ($V_H$III) umfasst:

das Substituieren von zumindest einem der Reste 4 und 71 in der $V_L$-Domäne durch eine andere Aminosäure; und
das Substituieren von zumindest drei der Reste 37, 67, 69, 71, 73, 75, 76, 78 und 94 in der $V_H$-Domäne durch eine andere Aminosäure,

worin die Nummerierung der Reste gemäß der Kabat-Nummerierung wie in Fig. 1 gezeigt gestaltet ist.

**13.** Verfahren nach Anspruch 12, worin das menschliche Gerüst vor Substitution das Gerüst der $V_L\kappa$-Untergruppe I ($V_L\kappa$I) aus Seq.-ID Nr. 7 und der $V_H$-Untergruppe III ($V_H$III) aus Seq.-ID Nr. 10 umfasst.

**14.** Verfahren nach Anspruch 12 oder Anspruch 13, umfassend zumindest eine der folgenden Substitutionen: an $V_L$4, Leucin; und an $V_L$71 , Tyrosin; und zumindest drei der folgenden Substitutionen: an $V_H$37, Isoleucin; an $V_H$67, Threonin; an $V_H$69, Phenylalanin; an $V_H$71, Leucin; an $V_H$73, Threonin; an $V_H$75, Alanin; an $V_H$76, Serin; an $V_H$78, Valin; und an $V_H$94, Lysin.

**15.** Verfahren nach einem der Ansprüche 12 bis 14, worin die Reste 37, 78 und 94 der $V_H$-Domäne durch die Aminosäuren Isoleucin, Valin bzw. Lysin substituiert werden.

**16.** Verfahren nach einem der Ansprüche 12 bis 15, worin die Reste 71, 73, 75 und 76 durch die Aminosäuren Leucin, Threonin, Alanin bzw. Serin substituiert werden.

**17.** Verfahren nach einem der Ansprüche 12 bis 16, worin Rest 46 der $V_L$-Domäne durch die Aminosäure Valin substituiert wird.

**18.** Verfahren nach einem der Ansprüche 12 bis 16, worin die $V_L$-Domäne die in Seq.-ID Nr. 8 gezeigte Sequenz aufweist und die $V_H$-Domäne die in Seq.-ID Nr. 11 gezeigte Sequenz aufweist.

**19.** Verfahren nach einem der Ansprüche 12 bis 17, worin:

die $V_L$-Domäne die in Seq.-ID Nr. 8 gezeigte Sequenz aufweist, in der Rest 46, Leucin, durch Valin substituiert wird; und
die $V_H$-Domäne die in Seq.-ID Nr. 11 gezeigte Sequenz aufweist.

**20.** Verfahren nach einem der Ansprüche 12 bis 16, worin:

die $V_L$-Domäne die in Seq.-ID Nr. 8 gezeigte Sequenz aufweist, in der die Reste 4, Methionin, und 71, Tyrosin, durch Leucin bzw. Phenylalanin substituiert werden; und
die $V_H$-Domäne die in Seq.-ID Nr. 1-1 gezeigte Sequenz aufweist, in der Rest 67, Phenylalanin, durch Threonin substituiert wird.

**21.** Verfahren nach einem der Ansprüche 12 bis 15, worin:

die $V_L$-Domäne die in Seq.-ID Nr. 7 gezeigte Sequenz aufweist, in der Rest 71, Phenylalanin, durch Tyrosin substituiert wird; und
die $V_H$-Domäne die in Seq.-ID Nr. 10 gezeigte Sequenz aufweist, in der die Reste 37, Valin; 78, Leucin; und 94, Arginin; durch Isoleucin, Valin bzw. Lysin substituiert werden.

**22.** Verfahren nach einem der Ansprüche 12 bis 15, worin:

die $V_L$-Domäne die in Seq.-ID Nr. 7 gezeigte Sequenz aufweist, in der die Reste 4, Methionin, und 71, Phenylalanin, durch Leucin bzw. Tyrosin substituiert werden; und
die $V_H$-Domäne die in Seq.-ID Nr. 10 gezeigte Sequenz aufweist, in der die Reste 37, Valin; 78, Leucin; und 94, Arginin, durch Isoleucin, Valin bzw. Lysin substituiert werden.

**23.** Verfahren nach einem der Ansprüche 12 bis 15, worin:

die $V_L$-Domäne die in Seq.-ID Nr. 7 gezeigte Sequenz aufweist, in der Rest 4, Methionin, durch Leucin substituiert wird; und
die $V_H$-Domäne die in Seq.-ID Nr. 10 gezeigte Sequenz aufweist, in der die Reste 37, Valin; 67, Phenylalanin; 78, Leucin; und 94, Arginin, durch Isoleucin, Threonin, Valin bzw. Lysin substituiert werden.

**24.** Verfahren nach einem der Ansprüche 12 bis 17, weiters umfassend die Schritte:

des Offenlegens der $V_L$- und $V_H$-Domänen durch Substitutionen an einem Phagemiden;
des Bestimmens, ob sich VEGF an $V_L$- und $V_H$-Domänen durch Substitutionen bindet; und
des Selektierens humanisierter Antikörper, die sich an VEGF binden.

**25.** Humanisierter Antikörper nach einem der Ansprüche 1 bis 11, der ein Antikörperfragment ist.

**26.** Antikörperfragment nach Anspruch 25, das ein Fab-, $Fab^1$-, $F(ab^1)_2$- oder Fv-Fragment ist.

**27.** Verfahren nach einem der Ansprüche 12 bis 24, worin der humanisierte Antikörper ein Antikörperfragment ist.

**28.** Verfahren nach Anspruch 27, worin das Fragment ein Fab-, $Fab^1$-, $F(ab^1)_2$- oder Fv-Fragment ist.

**29.** Humanisierter Antikörper nach Anspruch 1, für den ein Nucleinsäuremolekül mit der in Seq.-ID Nr. 14 gezeigten Sequenz kodiert.

**30.** Verwendung des humanisierten Antikörpers nach einem der Ansprüche 1 bis 11, 25, 26 und 29 bei der Herstellung eines Medikaments zur Verwendung bei der Inhibition von Tumorwachstum durch Inhibition mitogener Signalgebung.

## Revendications

**1.** Anticorps d'un fa cteur de croissance endothélial anti-vasculaire humanisé, où les régions déterminant la complémentarité (CDR) d'un anticorps non-humain sont greffées sur une charpente humaine comprenant la charpente du sous -groupe I de $V_L$k ($V_L$kI) de SEQ ID NO: 7 et la charpente du sous-groupe III de $V_H$ ($V_H$III) de SEQ ID NO: 10, où au moins l'un des résidus représentativement numérotés 4 et 71 du domaine de $V_L$ est substitué par un acide aminé différent et au moins trois des résidus représentativement numérotés 37, 67, 69, 71, 73, 75, 76, 78 et 94 du domaine de $V_H$ sont substitués par un acide aminé différent, et où le résidu 46 du domaine de $V_L$ est facultativement substitué par l'acide aminé valine, et où la numérotation des résidus est en accord avec la numérotation de Kabat telle que montrée à la Figure 1.

**2.** Anticorps humanisé de la revendication 1, ayant au moins l'une des substitutions qui suivent: à $VL_4$, leucine; et à $V_L$71, tyrosine; et au moins trois des substitutions qui suivent: à $V_H$37, isoleucine; à $V_H$67, thréonine; à $V_H$69, phénylalanine; à $V_H$71, leucine; à $V_H$73, thréonine; à $V_H$75, alanine; à $V_H$76, sérine; à $V_H$78, valine; et à $V_H$94, lysine.

**3.** Anticorps humanisé de la revendication 1 ou de la revendication 2, où les résidus 37, 78 et 94 du domaine de $V_H$

sont substitués par les acides aminés isoleucine, valine et lysine, respectivement.

**4.** Anticorps humanisé de toute revendication précédente, où les résidus 71, 73, 75 et 76 du domaine de $V_H$ sont substitués par les acides aminés leucine, thréonine, alanine et sérine, respectivement.

**5.** Anticorps humanisé de toute revendication précédente, où le résidu 46 du domaine de $V_L$ est substitué par l'acide aminé valine.

**6.** Anticorps humanisé de l'une quelconque des revendications 1 à 4, où le domaine de $V_L$ a la séquence indiquée à SEQ ID NO: 8 et le domaine de $V_H$ a la séquence indiquée à SEQ ID NO: 11.

**7.** Anticorps humanisé de l'une quelconque des revendications 1 à 5, où:

le domaine de $V_L$ a la séquence indiquée à SEQ ID NO: 8, dans laquelle le résidu 46, leucine, est substitué par la valine et
le domaine de $V_H$ a la séquence indiquée à SEQ ID NO: 11.

**8.** Anticorps humanisé de l'une quelconque des revendications 1 à 4, où:

le domaine de $V_L$ a la séquence indiquée à SEQ ID NO: 8, dans laquelle les résidus 4, méthionine, et 71, tyrosine, sont substitués par leucine et phénylalanine, respectivement; et
le domaine de $V_H$ a la séquence indiquée à SEQ ID NO: 11, dans laquelle le résidu 67, phénylalanine, est substitué par thréonine.

**9.** Anticorps humanisé de l'une quelconque des revendications 1 à 4, où:

le domaine de $V_L$ a la séquence indiquée à SEQ ID NO: 7, dans laquelle le résidu 71, phénylalanine, est substitué par tyrosine; et
le domaine de $V_H$ a la séquence indiquée à SEQ ID NO: 10, dans laquelle les résidus 37, valine; 78, leucine; et 94, arginine; sont substitués par isoleucine, valine et lysine, respectivement.

**10.** Anticorps humanisé de l'une quelconque des revendications 1 à 4, où:

le domaine de $V_L$ a la séquence indiquée à SEQ ID NO: 7, dans laquelle les résidus 4, méthionine et 71, phénylalanine, sont substitués par leucine et tyrosine, respectivement; et
le domaine de $V_H$ a la séquence indiquée à SEQ ID NO: 10 dans laquelle les résidus 37, valine; 78, leucine; et 94, arginine; sont substitués par isoleucine, valine et lysine, respectivement.

**11.** Anticorps humanisé de l'une quelconque des revendications 1 à 4, où:

le domaine de $V_L$ a la séquence indiquée à SEQ ID NO: 7, dans laquelle le résidu 4, méthionine, est substitué par leucine; et
le domaine de $V_H$ a la séquence indiquée à SEQ ID NO: 10, dans laquelle les résidus 37, valine; 67, phénylalanine; 78, leucine; et 94, arginine, sont substitués par isoleucine, thréonine, valine et lysine, respectivement.

**12.** Méthode d'humanisation d'un anticorps d'un facteur de croissance endothélial anti-vasculaire non -humain, comprenant les étapes de:

greffer des régions déterminant la complémentarité (CDR) d'un anticorps non -humain sur une charpente humaine comprenant le sous-groupe I de ($V_Lk_I$) et le sous -groupe III de ($V_HIII$) ;
substituer au moins l'un des résidus 4 et 71 dans le domaine de $V_L$ par un acide aminé différent; et
substituer au moins trois des résidus 37, 67, 69, 71, 73, 75, 76, 78 et 94 dans le domaine de $V_H$ par un acide aminé différent,

où la numérotation des résidus est en accord avec la numérotation de Kabat telle que montrée à la Figure 1.

**13.** Méthode de la revendication 12, où la charpente humaine comprend, avant substitution, la charpente du sous-groupe I de $V_Lk$ ($V_LkI$) de SEQ ID NO:7 et le sous-groupe III de $V_H$ ($V_HIII$) de SEQ ID NO:10.

**14.** Méthode de la revendication 12 ou de la revendication 13, comprenant au moins l'une des substitutions suivantes: à $V_L$4, leucine; et à $V_L$71, tyrosine; et au moins trois des substitutions suivantes: à $V_H$37, isoleucine; à $v_H$67, thréonine; à $V_H$69, phénylalanine; à $V_H$71, leucine; à $V_H$73 thréonine; à $V_H$75, alanine; à $V_H$ 76, sérine; à $V_H$78, valine; et à $V_H$94, lysine.

**15.** Méthode de l'une quelconque des revendications 12 à 14, où les résidus 37, 78 et 94 du domaine de $V_H$ sont substitués par les acides aminés isoleucine, valine et lysine, respectivement.

**16.** Méthode de l'une quelconque des revendications 12 à 15, où les résidus 71, 73, 75 et 76 sont substitués par les acides a minés leucine, thréonine, alanine et sérine, respectivement.

**17.** Méthode de l'une quelconque des revendications 12 à 16, où le résidu 46 du domaine de $V_L$ est substitué par l'acide aminé valine.

**18.** Méthode de l'une quelconque des revendications 12 à 16, où le domaine de $V_L$ a la séquence indiquée à SEQ ID NO: 8 et le domaine de $V_H$ a la séquence indiquée à SEQ ID NO: 11.

**19.** Méthode de l'une quelconque des revendications 12 à 17, où:

le domaine de $V_L$ a la séquence indiquée à SEQ ID NO: 8, dans laquelle le résidu 46, leucine, est substitué par valine et le domaine de $V_H$ a la séquence indiquée à SEQ ID NO: 11.

**20.** Méthode de l'une quelconque des revendications 12 à 16, où:

le domaine de $V_L$ a la séquence indiquée à SEQ ID NO: 8, dans laquelle les résidus 4, méthionine, et 71, tyrosine, sont substitués par leucine et phénylalanine, respectivement; et
le domaine de $V_H$ a la séquence indiquée à SEQ ID NO: 11, dans laquelle le résidu 67, phénylalanine, est substitué par thréonine.

**21.** Méthode de l'une quelconque des revendications 12 à 15, où:

le domaine de $V_L$ a la séquence indiquée à SEQ ID NO: 7, dans laquelle le résidu 71, phénylalanine, est substitué par tyrosine; et
le domaine de $V_H$ a la séquence indiquée à SEQ ID NO: 10, dans laquelle les résidus 37, valine; 78, leucine; et 94, arginine; sont substitués par isoleucine, valine et lysine, respectivement.

**22.** Méthode de l'une quelconque des revendications 12 à 15, où:

le domaine de $V_L$ a la séquence indiquée à SEQ ID NO: 7, dans laquelle les résidus 4, méthionine et 71, phénylalanine, sont substitués par leucine et tyrosine, respectivement; et
le domaine de $V_H$ a la séquence indiquée à SEQ ID NO: 10, dans laquelle les résidus 37 valine; 78, leucine; et 94, arginine; sont substitués par isoleucine, valine et lysine, respectivement.

**23.** Méthode de l'une quelconque des revendications 12 à 15, où:

le domaine de $V_L$ a la séquence indiquée à SEQ ID NO: 7, dans laquelle le résidu 4, méthionine, est substitué par leucine; et
le domaine de $V_H$ a la séquence indiquée à SEQ ID NO: 10, dans laquelle les résidus 37, valine; 67, phénylalanine; 78, leucine; et 94, arginine, sont substitués par isoleucine, thréonine, valine et lysine, respectivement.

**24.** Méthode de l'une quelconque des revendications 12 à 17 comprenant de plus les étapes de:

présenter les domaines de $V_L$ et $V_H$ par des substitutions sur un phagémide:

déterminer si VEGF se liera aux domaines de $V_L$ et $V_H$ par des substitutions;
sélectionner des anticorps humanisés qui se lieront à VEGF.

**25.** Anticorps humanisé de l'une quelconque des revendications 1 à 11, qui est un fragment d'anticorps.

**26.** Fragment d'anticorps de la revendication 2 5, qui est un fragment Fab, Fab, $F(ab^1)_2$ ou Fv.

**27.** Méthode de l'une quelconque des revendications 12 à 24, où l'anticorps humanisé est un fragment d'anticorps.

**28.** Méthode de la revendication 27, où le fragment est un fragment Fab, $Fab^1$, $F(ab^1)_2$ ou Fv.

**29.** Anticorps humanisé de la revendication 1 codé par une molécule d'acide nucléique ayant la séquence indiquée à SEQ ID NO: 14.

**30.** Utilisation de l'anticorps humanisé de l'une quelconque des revendications 1 à 11, 25, 26 et 29 dans la fabrication d'un médicament à utiliser dans l'inhibition de la croissance d'une tumeur par inhibition de la signalisation mitogène.

## $V_L$ domain

```
                  10        20        30        40
A4.6.1    DIQMTQTTSSLSASLGDRVIISCSASQDISNYLNWYQQKP
                **      *    * *
hu2.0     DIQMTQSPSSLSASVGDRVTITCSASQDISNYLNWYQQKP

hu2.10    DIQMTQSPSSLSASVGDRVTITCSASQDISNYLNWYQQKP


                  50        60        70        80
A4.6.1    DGTVKVLIYFTSSLHSGVPSRFSGSGSGTDYSLTISNLEP
          **** *                        **    * *
hu2.0     GKAPKLLIYFTSSLHSGVPSRFSGSGSGTDFTLTISSLQP
                                        •
hu2.10    GKAPKLLIYFTSSLHSGVPSRFSGSGSGTDYTLTISSLQP


                  90       100
A4.6.1    EDIATYYCQQYSTVPWTFGGGTKLEIK
            *                *   *
hu2.0     EDFATYYCQQYSTVPWTFGQGTKVEIK

hu2.10    EDFATYYCQQYSTVPWTFGQGTKVEIK
```

## $V_H$ domain

```
                  10        20        30        40
A4.6.1    EIQLVQSGPELKQPGETVRISCKASGYTFTNYGMNWVKQA
           *   *  ** *   *** *  *              *
hu2.0     EVQLVESGGGLVQPGGSLRLSCAASGYTFTNYGMNWVRQA
                                              •
hu2.10    EVQLVESGGGLVQPGGSLRLSCAASGYTFTNYGMNWIRQA


                  50 a       60         70        80
A4.6.1    PGKGLKWMGWINTYTGEPTYAADFKRRFTFSLETSASTAYL
               * *                     * *** ** *
hu2.0     PGKGLEWVGWINTYTGEPTYAADFKRRFTISRDNSKNTLYL
                                         • •  •• •
hu2.10    PGKGLEWVGWINTYTGEPTYAADFKRRFTISLDTSASTVYL


            abc      90       100abcdef         110
A4.6.1    QISNLKNDDTATYFCAKYPHYYGSSHWYFDVWGAGTTVTVSS
           *** ***   * *  *                *  *
hu2.0     QMNSLRAEDTAVYYCARYPHYYGSSHWYFDVWGQGTLVTVSS
                          •
hu2.10    QMNSLRAEDTAVYYCAKYPHYYGSSHWYFDVWGQGTLVTVSS
```

*FIG._1*

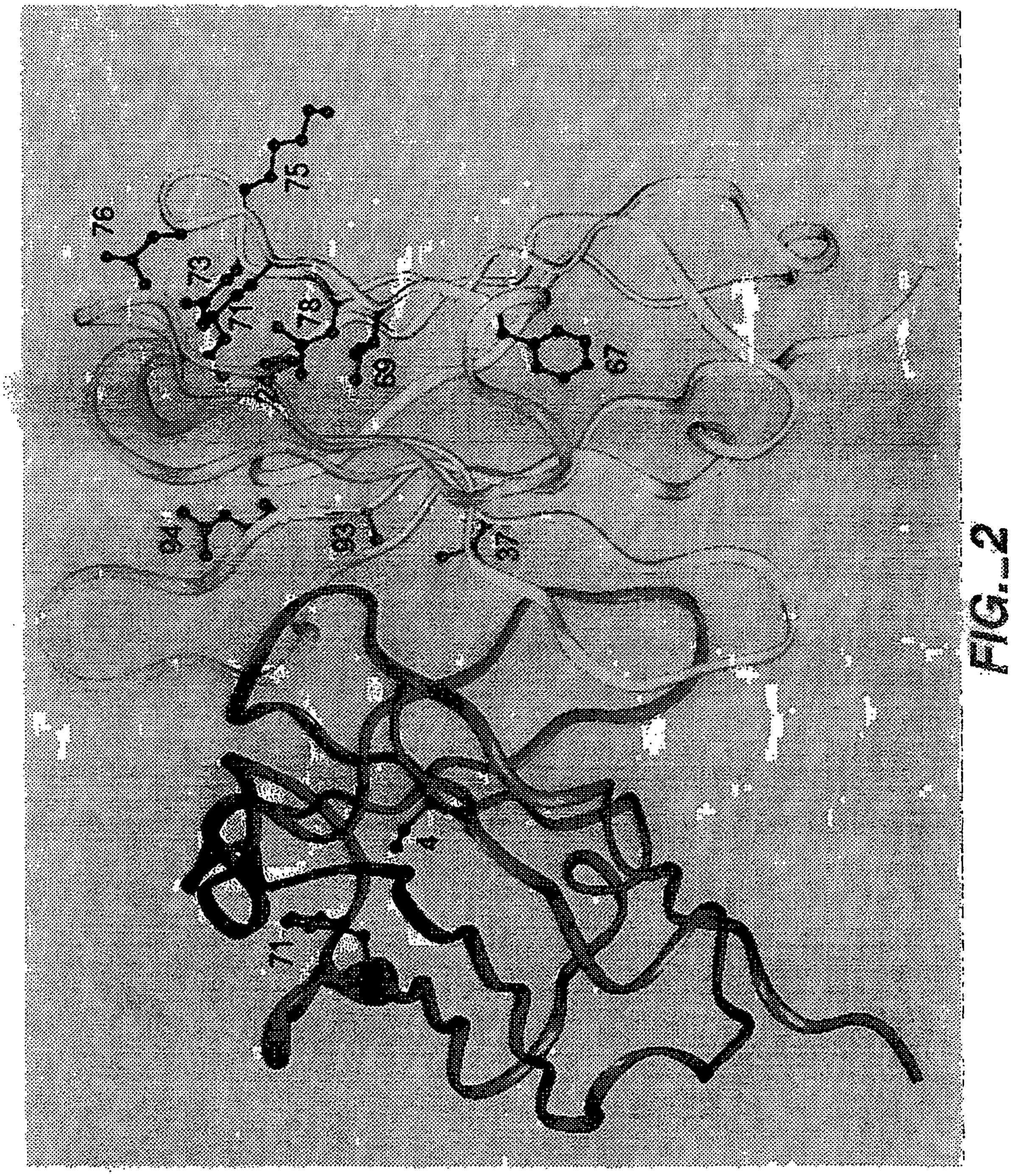


FIG._2

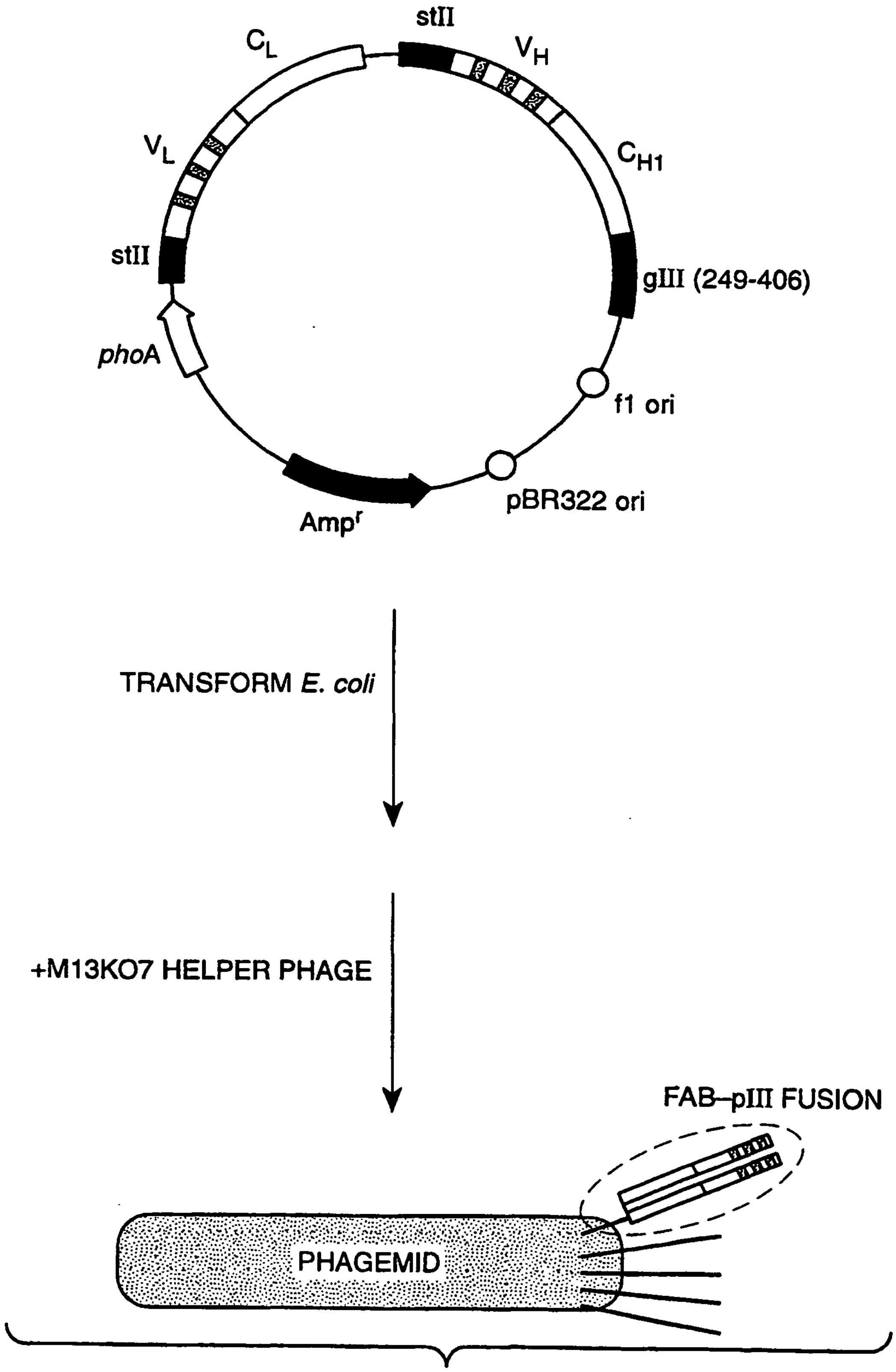
stII
$C_L$
$V_H$
$V_L$
$C_{H1}$
stII
gIII (249-406)
*pho*A
f1 ori
pBR322 ori
$Amp^r$
TRANSFORM *E. coli*
+M13KO7 HELPER PHAGE
FAB–pIII FUSION
PHAGEMID

FIG._3